# EUROPEAN PATENT APPLICATION

(11) **EP 4 260 876 A1**
(43) Date of publication of application: **18.10.2023**
(21) Application number: 22305549.2
(22) Date of filing: 14.04.2022
(51) Int. Cl.: A61K 49/22

(54) **COMPOSITIONS OF DISPERSED SYSTEMS FOR BIOMEDICAL APPLICATIONS, PROCESS FOR THEIR PREPARATION AND USES THEREOF**

(71) Applicant: Superbranche, 68420 Hattstatt (FR); Centre national de la recherche scientifique, 75016 Paris (FR)
(72) Inventor: FELDER-FLESCH, Delphine, 68420 HATTSTATT (FR); AYELA, Benjamin, 67400 ILLKIRCH GRAFFENSTADEN (FR); KRAFFT, Marie Pierre, 67000 STRASBOURG (FR); SHI, Da, 84570 MORMOIRON (FR); ABADIAN, Hagop, 67200 STRASBOURG (FR); EL-YAHKLIFI, Salima, 67200 STRASBOURG (FR)
(74) Representative: IPAZ

(57) **Abstract**

The invention relates to the use of oligo(ethylene oxide) dendritic molecules for stabilizing fluorocarbon-based nanoemulsions, to fluorocarbon-based nanoemulsions comprising such dendritic molecules and to their uses for biomedical applications, in particular as contrast agents or oxygenating agent.

## Description

### TECHNICAL FIELD

The invention belongs to the field of dispersed systems for biomedical application.

More particularly, the invention relates to the use of specific dendritic molecules (dendrons) for stabilizing fluorocarbon-based nanoemulsions, to fluorocarbon-based nanoemulsions comprising such dendritic molecules and to their uses for biomedical applications, in particular as contrast agents.

### BACKGROUND

Gaseous microbubbles are currently the subject of intense research in the fields of medical diagnostics and therapy. In combination with various ultrasound technologies, microbubbles are used clinically for cardiovascular imaging and early detection of cancers. In this context, the potential of microbubbles for ultrasound diagnosis, therapy (delivery of therapeutic agents under focused ultrasound), therapeutic energy delivery (histotripsy, embolotherapy, sonothrombolysis and tissue ablation), and potentiation of oxygen-dependent cancer therapies (radio- and chemotherapy, dynamic photo(sono)therapy) is intensively studied. Other potential applications include cell therapy and the treatment of neurodegenerative diseases (Alzheimer's, Parkinson's) by crossing the blood-brain barrier.

The versatility of functionalizing the surface of microbubbles, which makes possible to graft and/or incorporate therapeutic agents, biomarkers, various nanoparticles that are themselves functional, photo(sono)sensitizers adding other diagnostic (e.g. fluorescence and photoacoustic) and therapeutic (e.g. photothermal) modalities, greatly increases the field of applications and has led to the evaluation of several theranostic platforms ensuring better diagnosis/guidance, and thus improving therapies.

For many of these applications, the micrometric size of the microbubbles, which confines them to the vascular system, and their short life span in the circulation are important limitations. In particular, microbubbles cannot penetrate tumor tissues. To overcome these issues, one approach consists in injecting nanometric droplets (a nanoemulsion) of a liquid fluorocarbon and then in vaporizing them with ultrasonic pulses once they have reached their target. This approach is particularly promising in the treatment of cancers. It has been shown that nanodroplets tend to accumulate in tumor tissues; the application of ultrasound can then vaporize the liquid fluorocarbon. The nanoemulsion droplets are thus converted into microbubbles that can be used as contrast agents when submitted to low acoustic power ultrasound. The generated microbubbles can also be destroyed by cavitation to deliver the incorporated therapeutic ingredient.

Fluorocarbon-based nanoemulsions usually comprise an aqueous continuous phase in which are dispersed nanodroplets of a liquid fluorocarbon, said nanodroplets being stabilized by an interfacial film of at least one surfactant. The surfactants commonly used are selected among long chain fluoroalkylated surfactants or phospholipids.

However, the use of long chain fluoroalkylated surfactants (C ≥ 7) is highly questionable since perfluoroalkyl substances (PFAS) tend to disseminate, bioaccumulate and persist in the environment, and some of these compounds are toxic (Marie Pierre Krafft and Jean G. Riess, "Per- and polyfluorinated substances (PFASs): Environmental challenges", Elsevier - Current Opinion in Colloid & Interface Science, 20 (2015) 192-212. Their production and use are now strictly regulated in the western countries and in Japan. In addition, the use of phospholipidic surfactants, optionally in admixture with other surfactants such as for example sodium dodecylsulfate (SDS) to stabilize fluorocarbon-based emulsions does not give entire satisfaction since they lead to emulsions in which the mean size of the dispersed droplets is of micrometric scale or to emulsions that do not generate stable gaseous microbubbles after ultrasonic activation.

Therefore, a major limitation to the development of activable microbubbles is the lack of surfactants specifically designed to 1) effectively stabilize fluorocarbon nanodroplets in fluorocarbon-based emulsions, 2) provide control of droplet activation into microbubbles, and 3) stabilize the microbubbles to avoid side effects such as pulmonary embolism, increase their intravascular persistence and facilitate diagnosis.

The inventors have set themselves the goal of developing a solution to overcome these drawbacks, in particular to obtain a stable fluorocarbon-based nanoemulsion which can be easily activated into stable microbubbles with a mean diameter not exceeding 2 to 3 µm.

A first objective of the present invention is to provide a class of dendritic molecules that can be used in fluorocarbon-based nanoemulsions or phase change emulsions (PCEs) that can be activated by various stimuli, including ultrasound or temperature, to generate stable microbubbles. These dendritic molecules are able to (i) control the size and stabilize the fluorocarbon nanodroplets, (ii) precisely control the phase change phenomenon, (iii) obtain microbubbles with predetermined sizes and size distributions, and (iv) stabilize these microbubbles. Depending on the nature of their substituents, some of these dendritic molecules are also able to be grafted on metallic oxide nanoparticles which are particularly useful as medical imaging tools, in particular as an optical imaging tool or magnetic resonance imaging (MRI) tool, more particularly MRI contrast agent, or as a hyperthermia and/or radiosensitizing agent for the treatment of tumors or other pathological tissues.

A second objective of the present invention is also to provide a class of dendritic molecules effective and useful in controlling the properties of nanoemulsions and microbubbles, independently of the phase change process.

These objectives are reached thanks to the use of specifically designed dendritic molecules of formula (I) as described in details thereafter.

### Description of the invention

A first object of the present invention is the use of an oligo(ethylene oxide) dendritic molecule of formula (I) below: 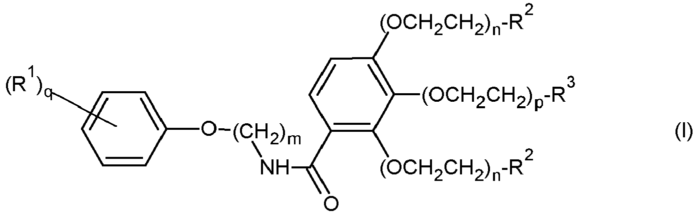 wherein :
- R¹ is a group selected among:
   ^{∗} an alkyl radical having at least 2 carbon atoms or an alkyl radical having at least 2 carbon atoms and comprising a terminal fluorinated group,
   ^{∗} a group -OR⁴ or -COOR⁴ in which R⁴ represents a linear alkyl radical having at least 4 carbon atoms or an alkyl radical having at least 2 carbon atoms and comprising a terminal fluorinated group, and
   ^{∗} a phosphonate group of the following formula (PG): 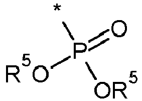 (PG) in which each of R⁵ represents a linear alkyl radical having at least 4 carbon atoms and the star represents the attachment point of said group of formula (PG) to the phenyl cycle;
- each of R² represents a linear alkyloxy radical having from 1 to 20 carbon atoms;
- R³ represents a linear alkyloxy radical having from 1 to 20 carbon atoms or a carboxyl group;
- n is an integer ranging from 1 to 16;
- p is an integer ranging from 1 to 16;
- m is an integer ranging from 1 to 4, preferably m = 1 or 2 and more preferably m = 2; and
- q is an integer ranging from 1 to 3, with the proviso that when R¹ represents a phosphonate group PG, then q = 2,
for stabilizing a fluorocarbon-based nanoemulsion consisting of a dispersion of nanodroplets of a liquid fluorocarbon stabilized in a continuous aqueous phase by a thin lipid film present at the interface between said aqueous phase and said liquid fluorocarbon, said lipid film comprising phospholipid(s) and at least one oligo(ethylene oxide) dendron of formula (I).

Thanks to these particularly designed dendritic molecules, it is now possible to accede to fluorocarbon-based nanoemulsions having an enhanced stability, which are easy to prepare, which does not involve the use of long-chain fluoroalkylated surfactants, and which can be easily activated into stable gaseous microbubbles not exceeding 2-3 µm in diameter and particularly useful in different biomedical applications.

More particularly, the oligo(ethylene oxide) dendritic molecules of formula (I) exhibit (1) a very good miscibility with phospholipid monolayers (widely used in the formulation of medical microbubbles), (2) a good affinity with the fluorocarbon encapsulated by the lipid film, especially when the dendritic molecules are fluorinated, and allow after controlled activation of the nanoemulsion by ultrasound or temperature (3) re-spreading of the interfacial film at the surface of the microbubbles by controlling the lateral interactions in the lipid monolayer, and finally 4) sufficient anchoring at the gas/water interface to ensure the stability of the microbubbles.

The innovation is therefore in obtaining microbubbles by controlled activation of stable fluorocarbon-based nanoemulsions with an intravascular persistence that is much longer than that of micron-sized microbubbles, which allows their accumulation in tumors. The oligo(ethylene) dendritic compounds represent a versatile platform onto which ligands can be grafted for targeted delivery, if needed.

In addition, the oligo(ethylene oxide) dendritic molecules of formula (I) in which q = 2 and R¹ represents a phosphonate group of formula PG may be grafted on magnetic nanoparticles, which combine the assets of magnetic nanoparticles, for example to allow a guidance (monitoring) during magnetic resonance imaging.

In the present description, the terms "oligo(ethylene glycol) dendritic molecule of formula (I)" and "dendron of formula (I)" are synonyms.

With reference to R¹, "linear alkyl radical having at least 2 carbon atoms" means a hydrocarbon group with a linear chain of at least 2 carbon atoms, preferably from 2 to 12 carbon atoms and more preferably from 2 to 8 carbon atoms. Examples of said groups are methyl, propyl, butyl, pentyl, hexyl, heptyl, octyl, nonyl, decyl, undecyl and dodecyl groups.

With reference to R¹ and R⁴, "alkyl radical having at least 2 carbon atoms and comprising a terminal fluorinated group" means a hydrocarbon group with a linear or branched chain of at least 2 carbon atoms, preferably from 2 to 12 carbon atoms, and more preferably from 2 to 8 carbon atoms, and bearing at the end of said chain, at least one fluorinated group. Non limitating examples of fluorinated groups are -CF₂-CF₃ and -CF(-CF₃)₂.

With reference to R⁴, "linear alkyl radical having at least 4 carbon atoms" means a hydrocarbon group with a linear chain of at least 4 carbon atoms, preferably from 4 to 12 carbon atoms and more preferably from 4 to 8 carbon atoms. Examples of said groups are butyl, pentyl, hexyl, heptyl, octyl, nonyl, decyl, undecyl and dodecyl groups.

With reference to R⁵ "linear alkyl radical having at least 1 carbon atom" means a hydrocarbon group with a linear chain of at least 1 carbon atom, preferably from 2 to 12 carbon atoms and more preferably from 2 to 8 carbon atoms. Examples of said groups are methyl, ethyl, propyl, butyl, pentyl, hexyl, heptyl, octyl, nonyl, decyl, undecyl and dodecyl groups.

With reference to R² and to R³, "linear alkyloxy radical having from 1 to 20 carbon atoms" means a hydrocarbon group with a linear chain of 1 to 20 carbon atoms, preferably from 1 to 4 carbon atoms, and more preferably having only one carbon atom, said linear chain of carbon atoms being linked to an oxygen atom. Examples of said group are methyloxy, ethyloxy, propyloxy and butyloxy groups.

According to a preferred embodiment of the present invention, n is an integer ranging from 4 to 6 inclusively and even more preferably n = 4.

According to another preferred embodiment of the present invention, p is an integer ranging from 4 to 10 inclusively and even more preferably p = 4 or p = 9.

According to a particular and preferred embodiment of the present invention, the oligo(ethylene oxide) dendritic molecule of formula (I) is a compound in which q = 2 and R¹ represents a phosphonate group of formula (PG) in which each of R⁵ represents a hydrogen atom or an alkyl group having from 4 to 12 carbon atoms, more preferably R⁵ is an alkyl group selected among octyl, decanyl, and dodecanyl. According to this particular embodiment, said oligo(ethylene oxide) dendritic molecule of formula (I) may be grafted on magnetic nanoparticles, preferably metallic oxide nanoparticles and even more particularly iron oxide nanoparticles. Always according to this particular embodiment, said nanoparticles are grafted thanks to the phosphonate groups (PG) to which they may adsorb or react to form a covalent bond.

As an example of metallic oxide nanoparticles, those used and disclosed in international application WO 2005/150502 may be mentioned.

According to another particular embodiment of the present invention, the oligo(ethylene oxide) dendritic molecule of formula (I) is a compound in which q = 2 and R¹ represents an alkyl group selected among octyl, decanyl, and dodecanyl or a fluorinated group selected among -(CH₂)₆-CF₂CF₃ and - (CH₂)₂-CF(CF₃)₂.

According to another particular embodiment of the present invention, the oligo(ethylene oxide) dendritic molecule of formula (I) is a compound in which q = 2 and R¹ represents a group -OR⁴ or -COOR⁴ in which R⁴ represents an alkyl group selected among octyl, decanyl, and dodecanyl or a fluorinated group selected among -(CH2)6-CF2CF3 and -(CH₂)₂-CF(CF₃)₂.

According to a particular and preferred embodiment of the present invention, the oligo(ethylene oxide) dendritic molecule of formula (I) is a compound in which each of R² represents a methyloxy group.

According to another particular and preferred embodiment of the present invention, the oligo(ethylene oxide) dendritic molecule of formula (I) is a compound in which R³ represents a methyloxy group or a carboxyl group.

According to a most preferred embodiment of the present invention, the oligo(ethylene oxide) dendritic molecule of formula (I) is a compound selected among compounds of formulae (I-A) to (I-Q) whose significations of R¹ to R⁵, m, n, p, and q are given in the following Table 1:

**TABLE 1**

| **Cpds** | **R¹** | **R²** | **R³** | **R⁴** | **R⁵** | **n** | **P** | **m** | **q** |
|---|---|---|---|---|---|---|---|---|---|
| (I-A) | PG | -OCH₃ | -OCH₃ | - | -(CH₂)₇-CH₃ | 4 | 4 | 2 | 2 |
| (I-B) | PG | -OCH₃ | -OCH₃ | - | -(CH₂)₉-CH₃ | 4 | 4 | 2 | 2 |
| (I-C) | PG | -OCH₃ | -OCH₃ | - | (CH₂)₁₁-CH₃ | 4 | 4 | 2 | 2 |
| (I-D) | PG | -OCH₃ | -COOH | - | -(CH₂)₇-CH₃ | 4 | 9 | 2 | 2 |
| (I-E) | PG | -OCH₃ | -COOH | - | -(CH₂)₉-CH₃ | 4 | 9 | 2 | 2 |
| (I-F) | PG | -OCH₃ | -COOH | - | -(CH₂)₁₁-CH₃ | 4 | 9 | 2 | 2 |
| (I-G) | -(CH₂)₇-CH₃ | -OCH₃ | -COOH | - | - | 4 | 9 | 2 | 2 |
| (I-H) | -(CH₂)₆CF₂CF₃ | -OCH₃ | -COOH | - | - | 4 | 9 | 2 | 2 |
| (I-I) | -(CH₂)₂CF(CF₃)₂ | -OCH₃ | -COOH | - | - | 4 | 9 | 2 | 2 |
| (I-J) | -COOR⁴ | -OCH₃ | -COOH | -(CH₂)₇-CH₃ | - | 4 | 9 | 2 | 2 |
| (I-K) | -COOR⁴ | -OCH₃ | -COOH | - (CH₂)₆CF₂C F₃ | - | 4 | 9 | 2 | 2 |
| (I-L) | -COOR⁴ | -OCH₃ | -COOH | - (CH₂)₂CF(C F₃)₂ | - | 4 | 9 | 2 | 2 |
| (I-M) | -OR⁴ | -OCH₃ | -COOH | -(CH₂)₇-CH₃ | - | 4 | 9 | 2 | 2 |
| (I-N) | -OR⁴ | -OCH₃ | -COOH | - (CH₂)₂CF(C F₃)₂ | - | 4 | 9 | 2 | 2 |
| (I-O) | -OR⁴ | -OCH₃ | -COOH | - (CH₂)₆CF₂C F₃ | - | 4 | 9 | 2 | 2 |
| (I-P) | PG | -OCH₃ | -COOH | -(CH₂)₇-CH₃ | H | 4 | 9 | 2 | 2 |
| (I-Q) | PG | -OCH₃ | -COO*t*Bu | | -CH₂CH₃ | 4 | 9 | 2 | 2 |

Among the compounds specifically recited in the above table 1, compounds of formulae (I-A), (I-B), (I-C), (I-D), (I-E), (I-P) and (I-Q) are most preferred.

The dendritic molecules of formula (I) in which R¹ is a phosphonate group of formula (PG) in which R⁵ has the same meaning as formula (I) above except a hydrogen atom, q = 2, n = p and R² and R³ are identical and represent an alkyloxy group as defined above in formula (I), can be prepared according to a process comprising at least one step of reacting a compound of formula (II) below: 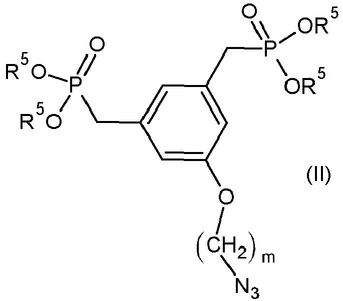
wherein R⁵ has the same meaning as formula (I) above except a hydrogen atom and m have the same signification as in formula (I) above, with a compound of formula (III) below: 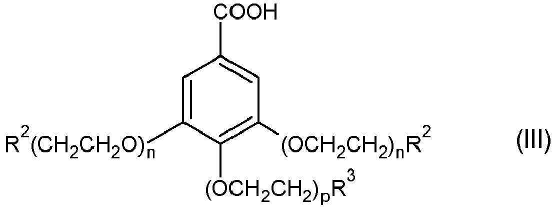
wherein n = p and have the same definition as in formula (I) above and further wherein R² and R³ are identical and represent an alkyloxy group as defined in formula (I), to lead to the corresponding compound of formula (I).

The reaction of compounds of formulae (II) and (III) can be carried out at room temperature, i.e. at a temperature ranging from 18 to 25°C, by mixing a solution of a compound of formula (II) in an appropriate solvent such as for example ethyl acetate, in the presence of a catalyst such as palladium/C, with a solution of a compound of formula (III) in an appropriate solvent such as for example dichloromethane in the presence of oxalyl chloride, dimethylformamide and N,N-diisopropylethylamine. The resulting compound of formula (I) can then be recovered and purified according to the usual practice known from one skilled in the art.

Compounds of formula (III) may be prepared according to a process comprising at least the following steps:
- reacting an alcohol of formula R⁶-(OCH₂CH₂)ₙOH (IV) in which R⁶ represents a linear alkyloxy radical having from 1 to 20 carbon atoms with tosyl chloride to obtain a compound of formula (VI) below: 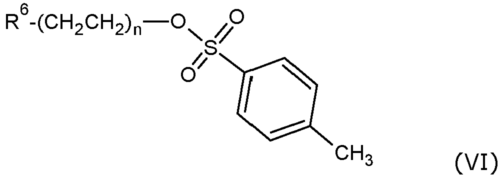 wherein R⁶ and n have the same meaning as in formula (III);
- reacting said compound of formula (VI) with methyl gallate to obtain a compound of formula (VIII) below: 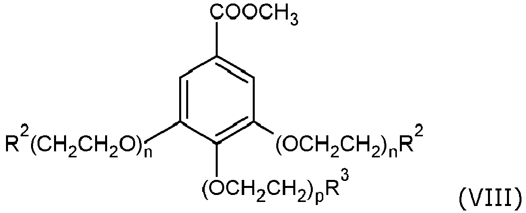 wherein n, p, R² and R³ have the same meaning as in formula (III); and
- unprotecting the carboxyl function of compound of formula (VIII) thus obtained to lead to the corresponding compound of formula (III).

The process for the preparation of compounds of formula (III) can be represented by the following Scheme 1:

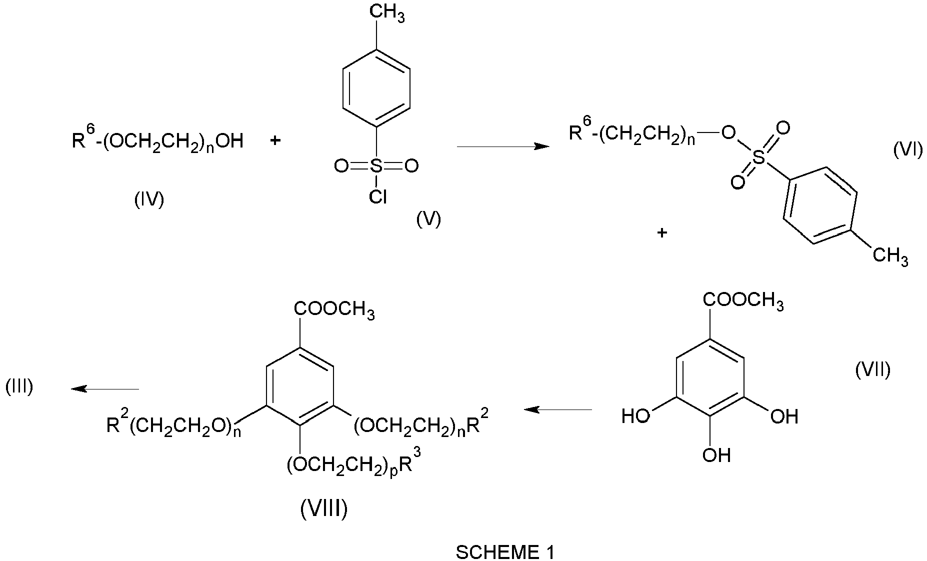

According to the process represented on Scheme 1, a solution of a compound of formula (IV) in which R⁶ has the same meaning as R² and R³ in the compounds of formula (III) above, (R² and R³ being identical) in an appropriate solvent such as for example dichloromethane in the presence of an amine such as for example triethylamine is contacted with a compound of formula (V), at room temperature under mixing until a compound of formula (VI) is obtained wherein R⁶ has the same meaning as in formula (IV) above, (R² and R³ being identical). The compound of formula (VI) is then reacted with a solution of compound of formula (VII) (methylgallate) in an appropriate solvent such as for example acetone, in the presence of potassium carbonate and potassium iodide and heated to reflux under mixing for about 8 to 16 hours to obtain a compound of formula (VIII) in which R² is identical to R³ and is an alkyl group as defined above in formula (I). The carboxyl group of compound of formula (VIII) is then unprotected by reacting said compound of formula (VIII) dissolved in an appropriate solvent such a lower alcohol, i.e. methanol or a mixture of a lower alcohol with water, in particular a mixture of methanol and water, in the presence of an alkalinizing agent such as for example sodium hydroxide, at room temperature, to lead to the corresponding compound of formula (III).

Compounds of formula (II) may be prepared according to a process comprising at least the following steps:
- reacting an alcohol of formula R⁵-OH (IX), wherein R⁵ has the same meaning as formula (I) above except a hydrogen atom, with trimethylphosphite (compound of formula (X)) to obtain a compound of formula (XI) below: 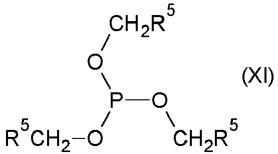 wherein R⁵ has the same meaning as formula (I) above except a hydrogen atom;
- reacting the compound of formula XI thus obtained with 3,5-bis(bromomethyl)phenol (compound of formula (XII)) to obtain a compound of formula (XIII) below: 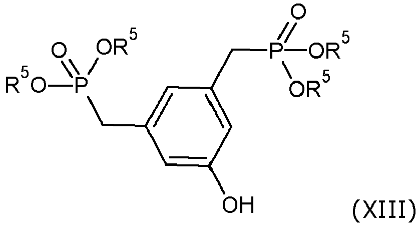 wherein R⁵ has the same meaning as formula (I) above except a hydrogen atom; and
- reacting the compound of formula (XIII) thus obtained with a compound of formula (XIV) below: 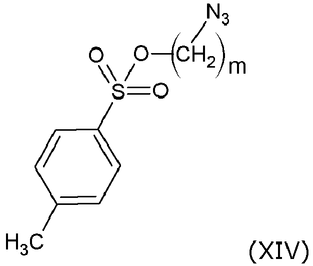 in which m has the same meaning as in formula (I) to obtain the corresponding compound of formula (II).

The process for the preparation of compounds of formula (II) can be represented by the following Scheme 2:

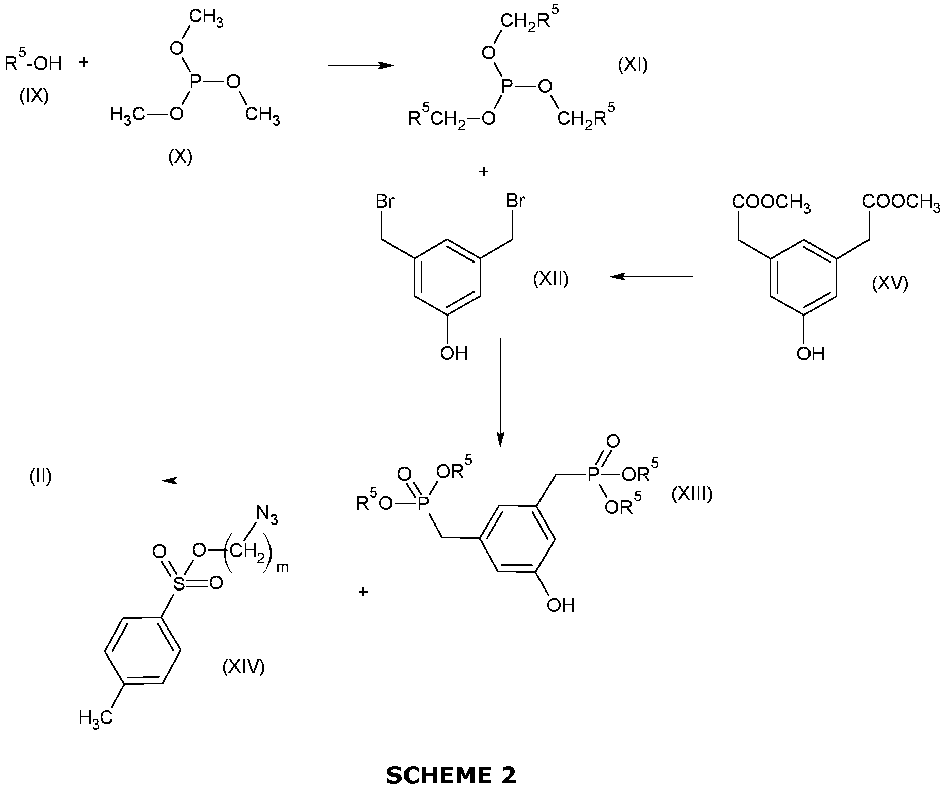

According to the process represented on Scheme 2, a compound of formula (X) (trimethylphosphite) is added to an alcohol of formula (IX) in which R⁵ has the same meaning as in formula (I) above, said alcohol of formula (IX) having previously been heated at a temperature of 30 to 75°C. The resulting mixture is then heated to a temperature of 130 to 230°C under argon atmosphere during 5 to 16 hours, to lead to compound of formula (XI) in which R⁵ has the same meaning as in formula (I) above. Compound (XI) may be separated from the remaining alcohol of formula (IX) for example by distillation. Compound of formula (XI) is then contacted with a compound of formula (XII) under stirring at a temperature of about 110 to 150°C for a period of time ranging from 8 to 16 hours to lead to compound of formula (XIII) in which R⁵ has the same meaning as formula (I) above except a hydrogen atom. A compound of formula (XIV) in which m has the same meaning as in formula (I) is added to a solution of the compound of formula (XIII) in an appropriate solvent such as for example toluene, said solution comprising an alkalinizing agent such as for example potassium hydroxide and potassium iodide and being previously heated at a temperature of 60 to 90°C. The resulting mixture is maintained at a temperature of 60 to 90°C and stirred for 8 to 16 hours to lead to a compound of formula (II) which can be recovered and purified by the usual techniques well known from one skilled in the art.

Compound of formula (XIV) can be previously prepared according to the process represented by the following Scheme 3:

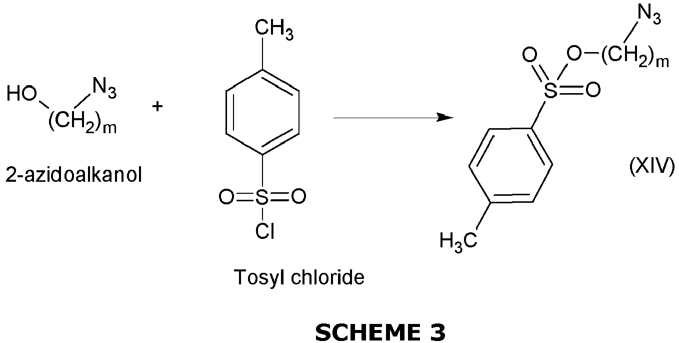

According to the process represented on Scheme 3, a solution of a 2-azidoalkanol in which m has the same meaning as in formula (I) in an appropriate solvent such as for example dichloromethane, is reacted with tosyl chloride in the presence of trimethylamine at room temperature for 8 to 16 hours to lead to a compound of formula (XIV) in which m has the same meaning as in formula (I).

The dendritic molecules of formula (I) in which R¹ is a phosphonate group (PG) wherein R⁵ has the same meaning as formula (I) above except a hydrogen atom, q = 2, n and p are identical or different and in which R² is an alkyloxy group as defined above in formula (I) and R³ is a carboxyl group, can be prepared according to a process comprising at least the following steps:
- reacting methyl gallate (compound of formula (VII) as defined above) with benzyl bromide to obtain a compound of formula (XV) below: 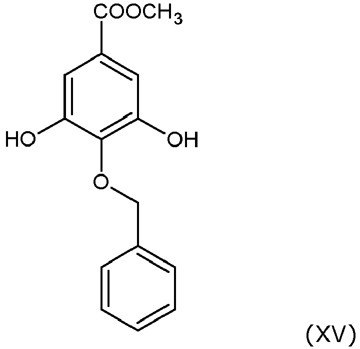
- independently reacting an alcohol of formula R²-(CH₂CH₂)ₙOH (IV') wherein R² and n have the same meaning as in formula (I) above with tosyl chloride (compound of formula (V) as defined above) to obtain a compound of formula (VI') below: 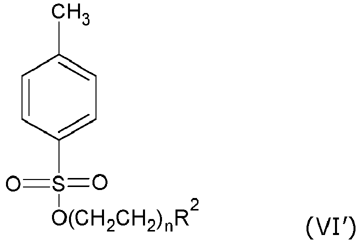 wherein R² and n have the same meaning as in formula (I) above;
- reacting the compound of formula (XV) and the compound of formula (VI') thus obtained to obtain a compound of formula (XVI) below: 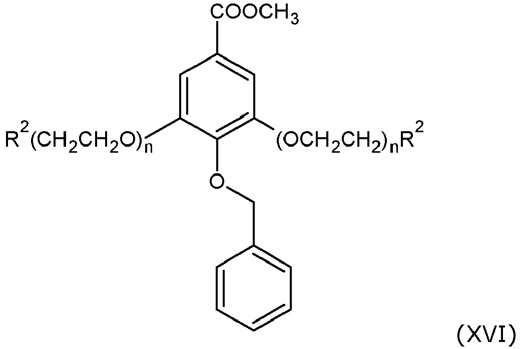 wherein R² and n have the same meaning as in formula (I) above;
- unprotecting the carboxylic function of the compound of formula (XVI) thus obtained to lead to the compound of formula (XVII) below: 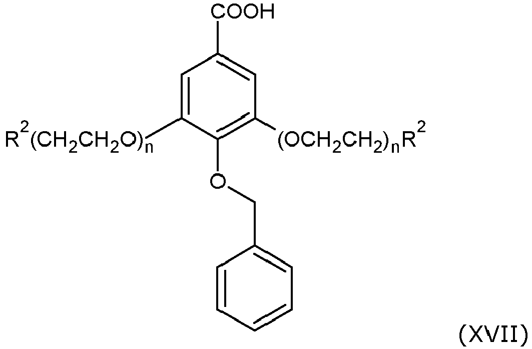 wherein R² and n have the same meaning as in formula (I) as defined above;
- reacting the compound of formula (XVII) thus obtained with a compound of formula (II) as defined above to obtain a compound of formula (XVIII) below: 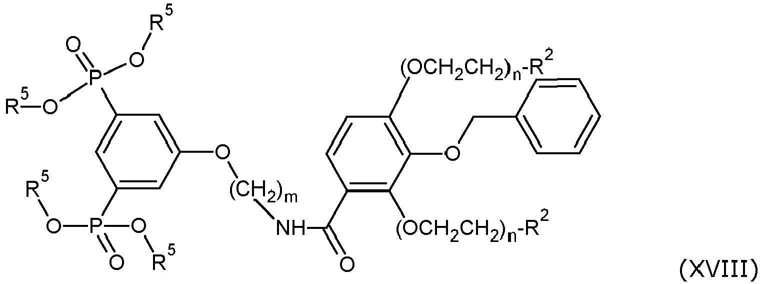 wherein R⁵ has the same meaning as formula (I) above except a hydrogen atom, and R², m and n have the same meaning as in formula (I) above;
- hydrolyzing the benzyl group of the compound of formula (XVIII) thus obtained to obtain a compound of formula (XIX) below: 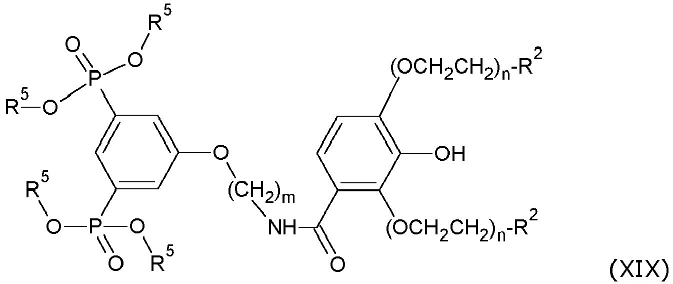 wherein R⁵ has the same meaning as formula (I) above except a hydrogen atom, and R², m and n have the same meaning as in formula (I) above;
- reacting the compound of formula (XIX) thus obtained with a compound of formula (XX) below: 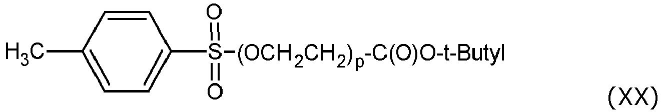
   wherein p has the same meaning as in formula (I) and has a value which is identical or different to the value of n of the compound in formula (XIX) as defined above, to obtain a compound of formula (XXI) below: 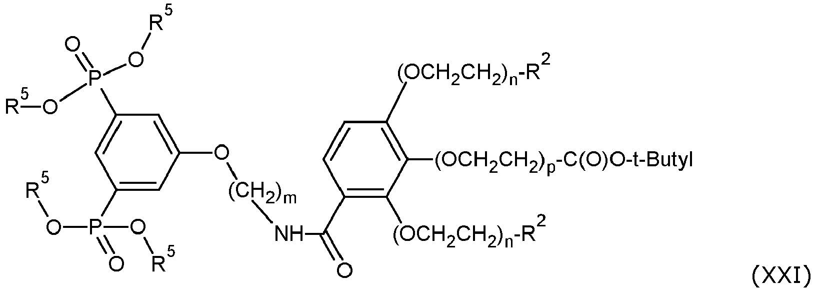
   wherein R⁵ has the same meaning as formula (I) above except a hydrogen atom, and R² m, n and p have the same meaning as in formula (I) above and p has the same meaning as in formula (I) and has a value which is identical or different to the value of n in the compound of formula (XIX) as defined above;
- unprotecting the carboxylic function of compound of formula (XXI) thus obtained to lead to the corresponding dendritic molecule of formula (I).

The process for the preparation of the dendritic molecules of formula (I) in which n and p are identical or different and in which R² is an alkyloxy group as defined above in formula (I) and R³ is a carboxyl group can be represented by the following Scheme 4:

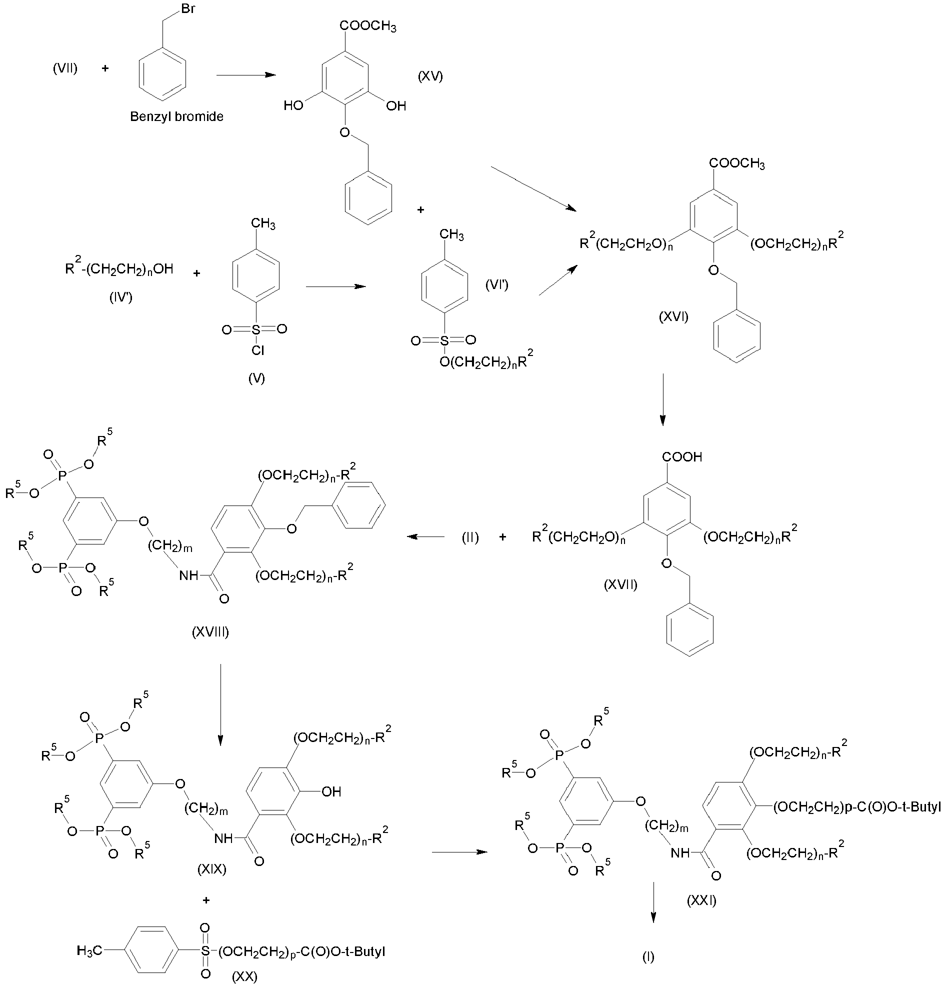

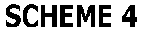

According to the process represented on Scheme 4, a compound of formula (VII) as defined above in Scheme 1 in solution in an appropriate solvent such as for example dimethylformamide is reacted with benzyl bromide in the presence of potassium hydrogen carbonate and potassium iodide at room temperature for 8 to 24 hours to lead to compound of formula (XV). Independently, a solution of a compound of formula (IV') in which R² as the same meaning as in formula (I) above, in an appropriate solvent such as for example dichloromethane in the presence of an amine such as for example trimethylamine, is contacted with a compound of formula (V), at room temperature under mixing until a compound of formula (VI') wherein R² as the same meaning as in formula (I) above is obtained. A solution of the compound of formula (XV) in an appropriate solvent such as for example acetone is then reacted with compound of formula (VI') thus obtained in the presence of an alkalinizing agent such as for example potassium carbonate, and potassium iodide. The resulting mixture is then heated at reflux for 8 to 24 hours to lead to the corresponding compound of formula (XVI) wherein R² and n have the same meaning as in the compound of formula (VI'). The carboxyl group of compound of formula (XVI) thus obtained is then unprotected by reacting said compound of formula (XVI) dissolved in an appropriate solvent such a lower alcohol, i.e. methanol or a mixture of a lower alcohol with water, in particular a mixture of methanol and water, in the presence of an alkalinizing agent such as for example sodium hydroxide, at room temperature, to lead to the corresponding compound of formula (XVII) wherein R² and n have the same meaning as in the compound of formula (XVI). The reaction of compounds of formula (II) as defined above with reference to the process represented in Scheme 2 and in which the radicals R⁵ have the same meaning as formula (I) above except a hydrogen atom and m has the same meaning as in formula (I) with the compound of formula (XVII) thus obtained can be carried out at room temperature, by mixing a solution of said compound of formula (II) in an appropriate solvent such as for example ethyl acetate, in the presence of a catalyst such as palladium/C, with a solution of a compound of formula (XVII) in an appropriate solvent such as for example dichloromethane in the presence of oxalyl chloride, dimethylformamide and then of N,N-diisopropylethylamine to lead to the corresponding compound of formula (XVIII) in which R⁵ has the same meaning as formula (I) above except a hydrogen atom, and R², m and n have the same meaning as in formula (I). A solution of the resulting compound of formula (XVIII) in an appropriate solvent such as for example ethyl acetate comprising a catalyst such as for example palladium/C is then purged with a hydrogen atmosphere in stirred at room temperature for 5 to 24 hours to lead to the corresponding compound of formula (XIX) in which R⁵ has the same meaning as formula (I) above except a hydrogen atom, and R² m and n have the same meaning as in formula (I). A solution of the compound of formula (XIX) thus obtained in an appropriate solvent such as for example acetone is then contacted with a compound of formula (XX) in which p has the same meaning as in formula (I) above, the value of p being equal of different to the value of n in the compound of formula (XIX), in the presence of potassium carbonate and potassium iodide. The resulting mixture is then heated at reflux for 8 to 24 hours to lead to the corresponding compound of formula (XXI) in which R⁵ has the same meaning as formula (I) above except a hydrogen atom, and R², m, n and p are as defined previously. The resulting compound of formula (XXI) can then be unprotected by addition of a strong acid, such as for example trifluoroacetic acid, into a solution of said compound of formula (XXI) in an appropriate solvent such as for example dichloromethane, and stirring at room temperature for 1 to 2 hours to lead to the expected corresponding compound of formula (I) which can then be recovered and purified according to the usual practice known from one skilled in the art.

Compounds of formula (XX) can be prepared according to a process comprising the step of reacting tosyl chloride with a compound of formula (XXII): OHCH₂CH₂-(OCH₂CH₂)ₚ₋₁-C(O)O-*t*-Butyl wherein p has the same meaning as in formula (I) above.

This process can be represented by the following Scheme 5:

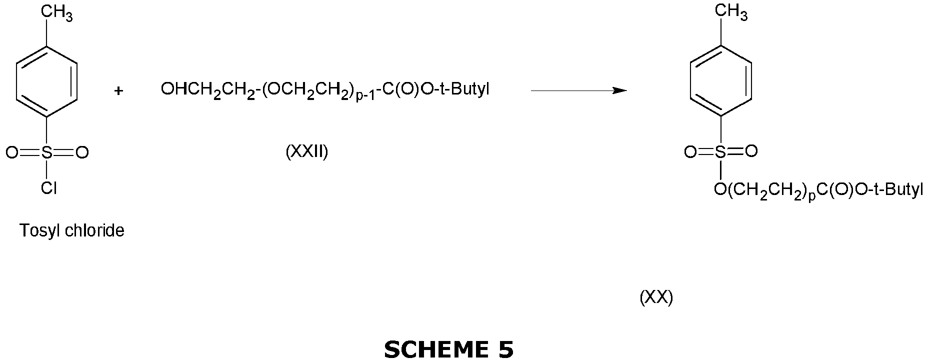

According to the process represented by Scheme 5, tosyl chloride is reacted with a solution of a compound of formula (XXII) in which p has the same meaning as in the compound of formula (I) and wherein p can have the same value than n or a different value than n, in an appropriate solvent such as for example dichloromethane, in the presence of an amine such as for example trimethylamine at room temperature for 8 to 24 hours. Compound of formula (XXII) thus obtained can then be recovered and purified by the technics well known by one skilled in the art.

The grafting of metallic oxide nanoparticles on compounds of formula (I) in which q = 2 and R¹ represents a phosphonate group (PG) can be then carried out according to the process described in international application WO 2015/150502.

The compounds of formula (I) in which R¹ is
^{∗} an alkyl radical having at least 2 carbon atoms or an alkyl radical having at least 2 carbon atoms and comprising a terminal fluorinated group; or
^{∗} a group -OR⁴ or -COOR⁴ in which R⁴ represents a linear alkyl radical having at least 4 carbon atoms or an alkyl radical having at least 2 carbon atoms and comprising a terminal fluorinated group,
can be prepared according to a process comprising the step of reacting a compound of formula (II'): 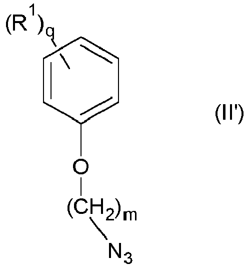
wherein m is as defined in formula (I) and R¹ is an alkyl radical having at least 2 carbon atoms or an alkyl radical having at least 2 carbon atoms and comprising a terminal fluorinated group, or a group -OR⁴ or -COOR⁴ in which R⁴ represents a linear alkyl radical having at least 4 carbon atoms or an alkyl radical having at least 2 carbon atoms and comprising a terminal fluorinated group, and q is an integer ranging from 1 to 3, with a compound of formula (III) as described above when, in the desired compound of formula (I), n and p are identical or different and R² is identical to R³, or with a compound of formula (XVII) as described above when, in the desired compound of formula (I), n and p are identical or different and R² is an alkyloxy group as defined above in formula (I) and R³ is a carboxyl group.

The conditions previously described for reacting a compound of formula (II) with a compound of formula (III) or with a compound of formula (XVII) also apply respectively to the reaction of a compound of formula (II') with a compound of formula (III) or with a compound of formula (XVII).

Compounds of formula (II') which would not be commercially available can be prepared by analogy according to methods well known by one skilled in the art and described for example in "Spacing-dependent dipolar interactions in dendronized magnetic iron oxide nanoparticle 2D arrays and powders." Solenne Fleutot, et al., Nanoscale, 2013, 5, 1507.

The compounds of formula (I) in which R¹ represents a PG group in which R⁵ represents a hydrogen atom can be prepared as described in international application WO2015150502.

A second object of the present invention is a fluorocarbon-based nanoemulsion comprising an aqueous continuous phase and a dispersion of nanodroplets consisting of a membrane of a lipidic phase encapsulating at least one liquid fluorocarbon, wherein the lipidic phase comprises at least one phospholipid and at least one oligo(ethylene oxide) dendritic molecule of formula (I) as defined above according to the first object of the present invention.

As used therein, the term "nanoemulsions" refers to emulsions of nanodroplets in aqueous media. The term "nanodroplets" refers submicron droplets comprising a liquid fluorocarbon.

As used therein, the term "fluorocarbon" (FC) refers to substances wherein the majority of or all the carbon-hydrogen bonds are replaced by carbon-fluorine bonds.

FC may be substituted, e.g. by halogen atoms such as bromine.

The FC may comprise a linear or branched fluorocarbon chain ranging in carbon length from 4 to about 10 carbon atoms.

Useful FCs include perfluorobutane, perfluoropentane, 2H,3H-perfluoropentane, perfluorohexane, perfluoroheptane, perfluorooctane, perfluorononane, perfluorodecalin, perfluorooctylbromide, and perfluorotripropylamine Preferred PFCs include perfluoropentane, 2H,3H-perfluoropentane, perfluorohexane, and perfluorooctylbromide. Most preferred is perfluorohexane.

The concentration of the FC in the nanoemulsion according to the present invention may vary from about 1 to 30% w/w, preferably from about 2.5 to 20% w/w, and even more preferably from about 5 to 10% w/w.

As used herein, "phospholipids" refers to a class of lipids whose molecule has a hydrophilic head containing a phosphate group and two hydrophobic chains derived from fatty acids, joined by an alcohol residue (usually a glycerol molecule).

Useful phospholipids according to the invention may have any suitable carbon chain length, for example ranging from about 12 carbon atoms to about 18 carbon atoms (e.g. 12, 13, 14, 15, 16, 17, 18) in length. The phospholipid molecules can also comprise unsaturations. Examples of phospholipids useful according to the invention include phosphatidylcholine derivatives such as for example dimyristoylphosphatidylcholine, dipalmitoylphosphatidylcholine, and distearoylphosphatidylcholine.

The concentration of phospholipids in the nanoemulsion according to the present invention may vary from about 0.25 to 10% w/w, preferably from about 0.4 to 5% w/w, and even more preferably from about 0.6 to 2.5% w/w.

The concentration of the oligo(ethylene oxide) dendritic molecule of formula (I) in the final nanoemulsion according to the present invention may vary from about 0.07% to 0.7% w/w, preferably from about 0.11% to 0.50% w/w, and even more preferably from about 0.13% to 0.38% w/w.

Among the oligo(ethylene oxide) dendritic molecules of formula (I), nanoemulsions in which the lipid phase comprises a dendron of formulae (I-P), (I-Q), (I-D), (I-E), (I-A), (I-B) or (I-C) are particularly preferred.

According to a particular and preferred embodiment of the present invention, the phospholipid/oligo(ethylene oxide) dendritic molecule of formula (I) molar ratio varies from about 5:1 to 50:1, more preferably from about 7:1 to 30:1 and even more preferably from about 9:1 to 25:1.

The average mean diameter of the nanodroplets may vary from about 50 to 900 nm, preferably from about 150 to 600 nm, and even more preferably from about 200 to 400 nm.

This average mean diameter can be adjusted by varying the phospholipid/dendritic molecule ratio. It can also be adjusted by varying the preparation parameters such as the pressure of homogenization or the number of cycles of the nanoemulsion through the high-pressure homogenizer.

The aqueous phase of the nanoemulsion may comprise water, e.g. water for injection (WFI), a saline solution or a buffer solution such as phosphate-buffered saline (PBS) or a HEPES buffer solution.

The fluorocarbon-based nanoemulsions as defined according to the second object of the present invention, can be prepared by any method known by one-skilled in the art, in particular by a process comprising (i) dispersing the phospholipid and the oligo(ethylene oxide) dendritic molecule of formula (I) in an aqueous phase at an appropriate temperature to obtain a dispersion ; (ii) adding the liquid fluorocarbon to the dispersion obtained in (i) ; (iii) homogenizing the resulting mixture to obtain a dispersion of nanodroplets consisting of a membrane of lipid phase encapsulating at least one liquid fluorocarbon or perfluorocarbon, wherein the lipid phase comprises at least one phospholipid and at least one oligo(ethylene oxide) dendritic molecule of formula (I).

As used herein, "an appropriate temperature" refers to a temperature higher than the phospholipid transition temperature. Depending on the nature of the phospholipid present in the nanoemulsion, an appropriate temperature may vary from about 25°C to 80°C, preferably from about 25°C to 50°C.

The homogenization may be carried out with any appropriate device, such as tip sonication, a low-energy device and or a high-pressure homogenization device.

After step (iii), the resulting nanoemulsion can be centrifuged, filtered or subjected to any other desirable procedure prior to characterization and use.

The nanoemulsion defined according to the second object of the invention may be used in different biomedical applications, in particular as contrast agents.

A third object of the present invention, is a fluorocarbon-based nanoemulsion as defined according to the second object of the present invention, for its use in biomedical applications, in particular as contrast agents for example for bimodal diagnostic applications.

More particularly, the fluorocarbon-based nanoemulsions according to the invention may be used as/for:
- a contrast agent for echosonography,
- a bimodal contrast agent for echosonography and MRI in the case of nanoemulsions bearing metallic oxide nanoparticles and in particular iron oxide nanoparticles,
- ultrasound-guided and spatially confined delivery of therapeutics such as drugs or genetic material into a target area,
- an oxygenating agent for the treatment of hypoxia which is useful in oncology. This property is based on the fact that fluorocarbons are known to be excellent solvents of gases, and in particular of respiratory gases such as oxygen and carbon dioxide.

Other advantages and embodiments of the present invention are given by the following examples and figures annexed in which:

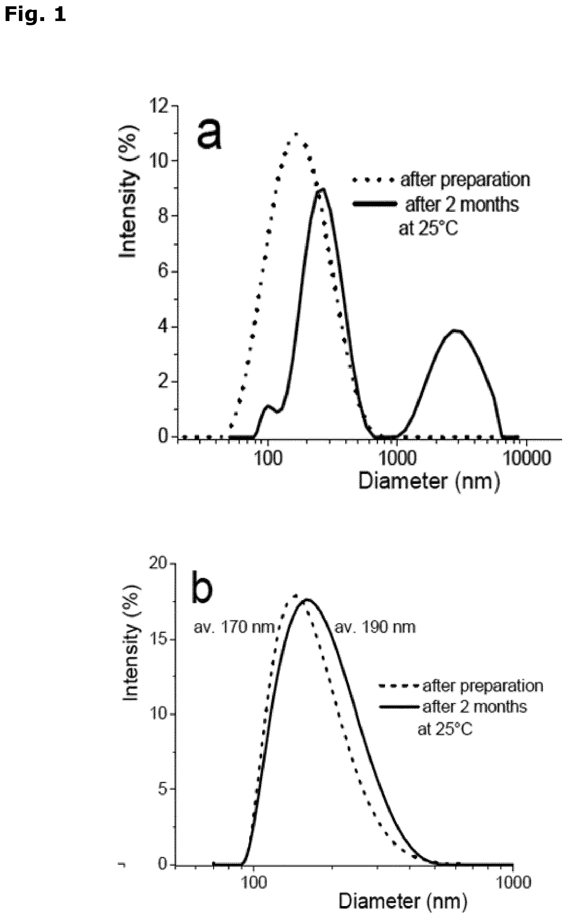

- Figure 1 is a graph representing the droplet size distribution in a reference nanoemulsion stabilized by dipalmitoylphosphatidylcholine only (figure 1a) and the droplet size distribution in a nanoemulsion according to the invention, i.e. stabilized by dipalmitoylphosphatidylcholine and a dendron of formula (I-P) (figure 1b). On figures 1a and 1b, intensity (%) is expressed as a function of diameter at t=0, i.e. just after the preparation of the nanoemulsions (dotted line curve) and after 2 months of storage at a temperature of 25°C (continuous line curve),

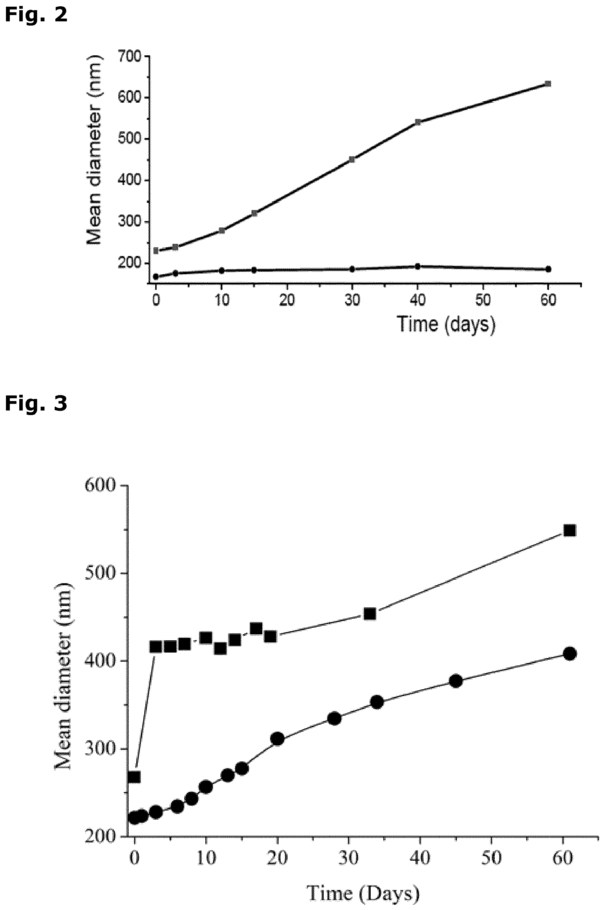

- Figure 2 is a graph representing the evolution of the average mean diameter of the droplets (in nm) as a function of time (in days) for the reference nanoemulsion stabilized by dipalmitoylphosphatidylcholine only (curve with the black squares) and for the nanoemulsion according to the invention, i.e. stabilized by dipalmitoylphosphatidylcholine and a dendron of formula (I-P) (curve with the black disks),
- Figure 3 is a graph representing the mean diameter of nanodroplets (in nm) as a function of time (in days) for a reference nanoemulsion not forming part of the present invention, i.e. containing only dimyristoylphosphatidylcholine (DMPC) (curve with black squares) comparatively to a nanoemulsion according to the invention, i.e. containing DMPC and a dendron of formula (I-P) (curve with black circles),

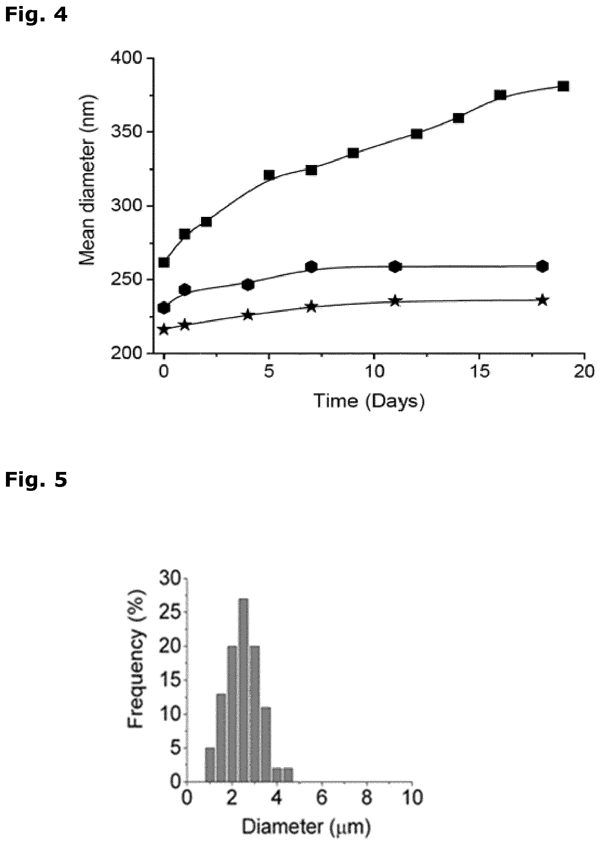

- Figure 4 is a graph representing the mean diameter of nanodroplets (in nm) as a function of time (in days) for a reference nanoemulsion not forming part of the present invention, i.e. containing only DMPC (square symbols) comparatively to a nanoemulsion according to the invention, i.e. containing DMPC and a dendron of formula (I-E) (circles) or to a nanoemulsion according to the invention, i.e. containing DMPC and a dendron of formula (I-D) (stars).
- Figure 5 is a graph representing the frequency (in %) as a function of the diameter of the microbubbles (in µm) of a microbubble dispersion obtained by activating a nanoemulsion stabilized with a dendritic compound of formula (I-P), just after its preparation;

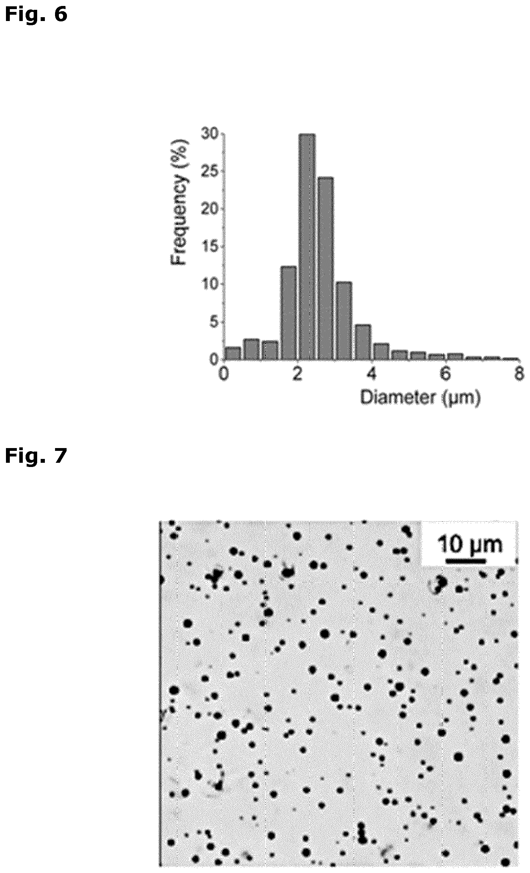

- Figure 6 is a graph representing the frequency (in %) as a function of the diameter of the microbubbles (in µm) of a microbubble dispersion obtained by activating a nanoemulsion stabilized with a dendritic compound of formula (I-P), after 7 hours at room temperature;
- Figure 7 is an optical photograph of the microbubble dispersion of figure 6,

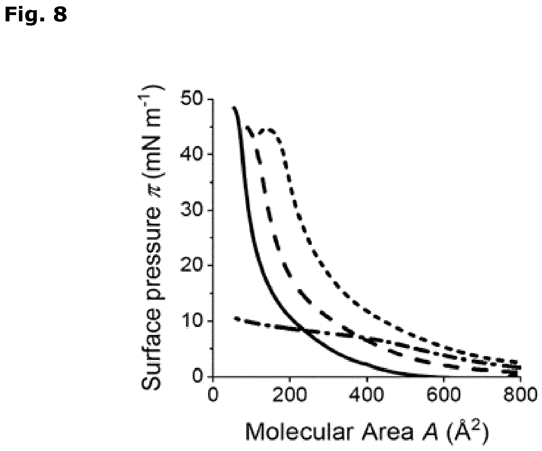

- Figure 8 is a graph representing the compression isotherms (surface pressure *π* (in mN m⁻¹) expressed as a function of molecular area A (in Å²)) of Langmuir monolayers formed by a dendritic compound of formula (I-A) (continuous line curve), a dendritic compound of formula (I-B) (curve with dashes), a dendritic compound of formula (I-C) (curve with the dotted lines) or a comparative dendritic compound of formula (DM) not forming part of the present invention (curve with alternating dashes and dotted lines).

### EXAMPLES

### EXAMPLE 1: Synthesis of a dendritic molecule of formula (I-A) according to the invention

In this example, a dendritic molecule of the following formula **(I-A)** was prepared:

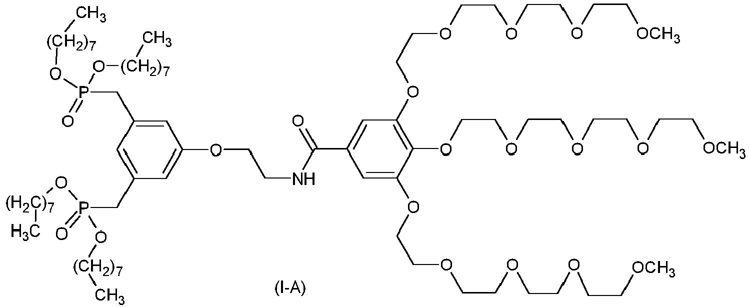

### 1.1 Step 1 - Preparation of 2,5,8,11-tetraoxatridecan-13-yl 4-methylbenzenesulfonate (compound 1)

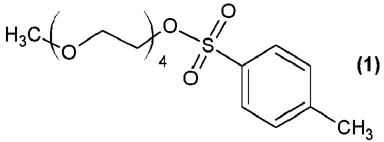

30.1 mmol (1 Eq.) of tetraethyleneglycol monomethyl ether were dissolved in 170.0 mL of dichloromethane at room temperature, then 5 mL of triethylamine (Et₃N) (36.2 mmol, 1.2 Eq.) were added and the reaction stirred for 10 min. 22.3 g (36.2 mmol, 1.2 Eq.) of tosyl chloride (solid) were slowly added. The resulting mixture was stirred at room temperature during the night.

A thin layer chromatography (TLC) analysis (stain phosphomolybdic acid (PMA) solution) showed the full consumption of PEG-OMe. The mixture was filtered on celite to remove the salts. The solvent was then evaporated under reduced pressure.

Purification by flash chromatography (100% dichloromethane (DCM) then 100% ethyl acetate (AcOEt)) afforded a clear liquid (10.1 g, 93%).

### Analysis:

¹H NMR (400 MHz, MeOD) δ 7.85 (d, *J* = 8.4 Hz, 2H), 7.50 (dd, *J* = 8.6, 0.8 Hz, 2H), 4.23 - 4.14 (m, 3H), 3.74 - 3.68 (m, 3H), 3.68 - 3.64 (m, 7H), 3.62 - 3.55 (m, 7H), 3.40 (s, 3H), 2.51 (s, 3H).

### 1.2 Step 2- Preparation of compound (2)

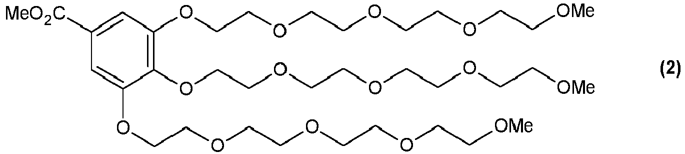

To a solution 8.8 mmol (1.0 Eq) of methyl gallate in acetone (60 mL) were added 18.1 mmol (3.2 Eq.) of potassium carbonate (K₂CO₃), 0.6 mmol (0.1 Eq.) of potassium iodide (KI) and 18.9 mmol (3.3 Eq.) of compound (1) as preparation in step 1 above. The resulting solution was heated to reflux for 42 hours.

The reaction mixture was cooled to room temperature, the solvent was removed, solid were suspended in dichloromethane (CH₂Cl₂), filtered over Celite, washed with an aqueous solution of sodium thiosulfate (Na₂S₂O₃) 2N and brine, dried over sodium sulfate (Na₂SO₄), filtered and concentrated under reduced pressure. Purification by flash chromatography (CH₂Cl₂/MeOH 98/2 to 96/4 to 9/1) gave 3.9g (89%) of a yellowish oil.

### Analysis:

¹H NMR (400 MHz, Chloroform-d) δ 7.46 (d, 2H: Ar*-H*)*,* 4.15 (dd, *J* = 5.6, 4.2 Hz, 6H: -O-C*H*₂-R), 3.86 (s, 3H: C*H*₃-O-C=O-R), 3.85 - 3.81 (m, 6H: -O-CH₂-C*H*₂-R), 3.70 - 3.66 (m, 6H: -O-C*H*₂-R), 3.63 - 3.56 (m, 24H: -O-C*H*₂-R), 3.51 - 3.47 (m, 6H: -O-C*H*₂-R), 3.33 (s, 9H: C*H*₃-O-).

### 1.3 Step 3 - Preparation of compound (3)

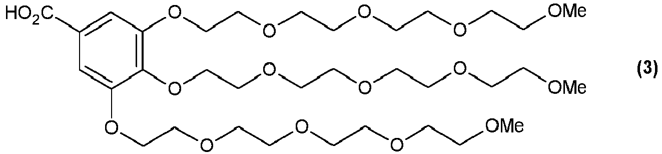

To a solution of 6.0 g (6.9 mmol - 1.0 Eq.) of compound **(2)** in methanol (MeOH) were successively added 1.1 g (34.5 mmol - 5.0 Eq.) of sodium hydroxide (NaOH) and 15.0 mL of distilled water. The yellow solution was stirred at room temperature (RT) overnight.

TLC analysis showed the consumption of compound **(2).** The solvents were removed by rotavap, then the crude product dissolved in CH₂Cl₂. HCl 2N (20.0 mL) was added and the stirring was continued for 15 min. The organic product was collected with CH₂Cl₂, the aqueous layer was washed with CH₂Cl₂ (five times), the combined organic layer was washed with brine, dried over Na₂SO₄, filtered and concentrated under reduced pressure to yield to compound **(3)** (5.1 g, 6.8 mmol, 99%) as yellow oil.

### Analysis:

¹H NMR (400 MHz, Chloroform-d) δ 7.46 (d, 2H: Ar-*H*), 4.15 (dd, *J* = 5.6, 4.2 Hz, 6H: -O-C*H*₂-R), 3.85 - 3.81 (m, 6H : -O-CH₂-C*H*₂-R), 3.70 - 3.66 (m, 6H : -O-C*H₂*-R), 3.63 - 3.56 (m, 24H : -O-C*H₂*-R), 3.51 - 3.47 (m, 6H : -O-C*H₂*-R), 3.33 (s, 9H : *CH3-O-).*

### 1.4 Step 4 - Preparation of 3,5-bis(bromomethyl)phenol (compound (4))

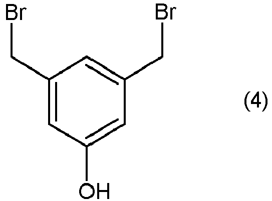

72 mL of lithium aluminum hydride 1M (LiAlH₄) (72 mmol - 1.8 Eq.) were dissolved in 150 mL of tetrahydrofuran (THF) at 0°C. Then 8.40 g (40 mmol - 1 Eq.) of dimethyl 5-hydroxyisophthalate were carefully added. The resulting solution was stirred at RT for 5 hours, then EtOAc (30.0 mL) and an aqueous solution of H₂SO₄ 10% (60.0 mL) were carefully added at 0°C. The stirring was continued overnight. An additional aqueous solution of H₂SO₄ 10% (60.0 mL) was added. The stirring was continued at RT for another 24h.

Once the aluminum salt was entirely dissolved, the aqueous layer was washed with EtOAc (at least 5 times). The combined organic layer was dried over Na₂SO₄, filtered and concentrated under reduced pressure to yield 8.3831 g of orange oil. The crude benzylic alcohol was then dissolved in 100 mL of acetic acid (AcOH) before 36 mL of hydrobromic acid (HBr 33% w/w in AcOH) (200 mmol - 5.0 Eq.) was carefully added at 0°C. The resulting mixture was stirred at RT for 2 days.

TLC analysis showed the full consumption of bis-benzylic alcohol, which is the intermediate formed but not isolated following the reduction of dimethyl 5-hydroxyisophthalate by LiAlH₄. The organic product was washed with brine (1 time), the aqueous layer was washed with EtOAc (at least five times), the combined organic layer was dried over Na₂SO₄, filtered and concentrated under reduced pressure. Purification by flash chromatography (CH₂Cl₂/EtOAc 1/0 to 96/4 to 9/1) afforded a yellowish solid. Recrystallization with a mixture of petroleum ether/diethyl oxide (EtP/Et₂O) afforded compound (4) (10.40 g, 37.4 mmol, 93.6%) as white solid.

### Analysis:

¹H NMR (400 MHz, Chloroform-d) δ 6.99 (s, 1H: Ar-*H*), 6.81 (d, *J* = 1.43 Hz, 2H: Ar-*H*), 4.41 (s, 4H: -CH*₂-*Ar).

### 1.5. Step 5 - Preparation of compound (5)

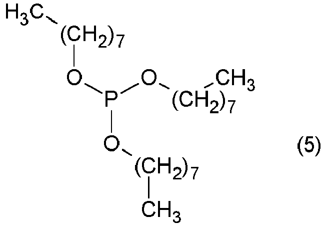

In a 100 mL two-necked flask connected to a Dean-Stark, 33.9 mL of octanol (107.25 mmol - 6 Eq.) were added, then the reaction setup was stirred and passed under a flow of argon for 10 min.

After that, the liquid was heated to 75°C and the flow replaced by 2 balloons of argon. 4.23 mL of trimethylphosphite (35.8 mmol - 1 Eq.) were added slowly from the syringe and then the setup was heated at 220 °C for 6 h.

Then the setup was cooled to ambient temperature, and the Dean-Stark containing the methanol formed during the reaction removed. An orange oil was obtained.

Distillation under reduced pressure was used to separate the remaining octanol from the compound **(5)** formed, at 160°C.

9.205 g of orange oil were recovered (yield = 61%)

### Analysis:

¹H-NMR (400MHz, Chloroform-d): δ 3.77 (q, J = 6.9 Hz, 6H, O-C*H*₂-R), 1.61 - 1.57 (m, 6H, O-C*H*₂-CH₂-R), 1.28 - 1.25 (m, 30H, C*H*₂), 0.86 (t, J = 6.7 Hz, 9H, C*H*₃-R).

31P-NMR (400MHz, CDCl₃): δ 139.15

### 1.6. Step 6 - Preparation of compound (6)

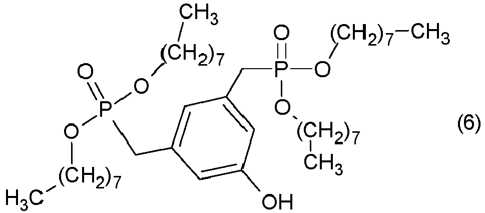

152 mg (0.543 mmol - 1 Eq.) of compound **(4)** and 916 mg (2.188 mmol - 4.0 Eq.) of compound **(5)** were added to a 50 mL flask. The reaction was stirred and heated to 140°C. overnight. A yellow-orange oil was obtained and a TLC was carried out (Petroleum ether / EtOAc 1/1):
The next day, after having cooled the solution, a Petroleum Ether / EtOAc chromatographic column was produced (1/1 to 2/3).

248 mg of compound **(6)** were recovered (yield = 64%).

### Analysis:

¹H NMR (400 MHz, CDCl₃) δ 6.81 (s, 2H, Ar*-H),* 6.64 (s, 1H, Ar*-H*)*,* 4.02 - 3.77 (m, 8H, P-C*H*₂), 3.28 - 2.81 (m, 4H, Ar-C*H*₂-P), 1.58 (m, 8H, P-CH₂-C*H*₂), 1.42 - 1.14 (m, 44H, C-C*H*₂), 0.95 - 0.77 (t, *J* = 7.1Hz, 12H, C-C*H*₃).

³¹P NMR (162 MHz, CDCl₃) δ 26.51.

### 1.7. Step 7 - Preparation of linker 2 (L2)

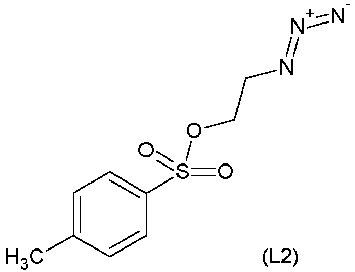

### 1.7.1. Step 7.1 - Preparation of linker 1 (L1)

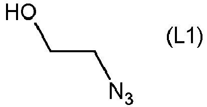

10.0 g (77.6 mmol - 1 Eq.) of 2-bromoethanol (97%) were dissolved in 12 mL of water, then at RT 6.12 g (93.2 mmol - 1.2 Eq.) of sodium azide (NaN₃) were added. The mixture was heated at 80°C for the night. Monitoring was made by TLC (DCM/MeOH, 95:5, SM Rf=0, EP Rf= 0,5).

After the night TLC showed just a few traces of the starting material, the mixture was stopped, then 10 mL of DCM was added. Phases were separated. Aqueous was extracted with 20 mL of DCM and NaCl solid was added at each extraction. The organic phase was directly engaged in the next step, without evaporation or further purification.

### 1.7.2. Step 7. - Preparation of linker 2 (L2)

To 6.76 g (77.6 mmol - 1 Eq.) of Linker 1 in DCM, were added 16.4 mL (116.0 mmol - 1.5 Eq.) of Et₃N slowly, the mixture was stirred 10 min, then 17.9 g (93.2 mmol - 1.2 Eq.) of tosyl chloride (TsCI) were added slowly.

After the night TLC (petroleum ether, AcOEt 7:3, KMnO₄) showed traces of the starting material. The mixture was concentrated, TsCI salts were precipitated in AcOEt and filtered over celite pad, washed 3 times with AcOEt, to give the desired linker 2 (22 g, brown oil).

It was then purified by Flash chromatography, Interchim-220g-30, liquid deposit in EtP/AcOEt (9:1), Eluent: 15 min EtP/AcOEt (9:1), 10 min EtP/AcOEt (8:2) with isocratic, and 10min EtP/AcOEt (7:3).
12.13 g of Linker 2 as a colourless oil was recovered.
¹H NMR (400 MHz, MeOD) δ 7.84 (d, *J* = 8.4 Hz, 2H), 7.49 (d, *J* = 7.8 Hz, 2H), 4.25 - 4.12 (m, 2H), 3.54 - 3.40 (m, 3H), 2.49 (s, 3H).

### 1.8. Step 8 - Preparation of compound (7)

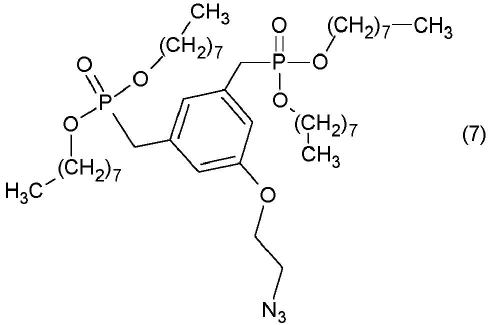

In a 25 mL flask were added 297.0 mg (1.23 mmol - 1.8 Eq.) of compound **(6),** 57.2 mg (1.02 mmol - 1.5 Eq.) of potassium hydroxide (KOH) and 11.3 mg (0.068 mmol - 0.1 Eq.) of KI in 15 mL of toluene. The solution was stirred for 20 min and heated to 60°C. to dissolve the reagents. 500.0 mg (0.684 mmol - 1.0 Eq.) of linker 2 (as prepared above in step 1.7. of example 1) was added and the reaction was left to stir overnight.

The flask was cooled to RT, then the toluene was evaporated off under reduced pressure. The crude as then dissolved in DCM, and filtered through Celite. A chromatographic column was carried out DCM / MeOH (1/0 to 98/2 to 96/4). 530 mg of compound **(7)** (97%) were thus recovered.

### Analysis:

¹H NMR (400 MHz, MeOD) δ 6.90 - 6.82 (m, 3H, Ar-*H*), 4.17 (t, *J* = 4.9 Hz, 2H, O-C*H*₂-CH₂-N₃), 3.98 (m, 8H, P-O-C*H*₂), 3.59 (t, *J* = 4.8 Hz, 2H, O-CH₂-C*H*₂-N₃), 3.27 - 3.16 (BX, *J* = 21.9 Hz, 4H, Ar-C*H*₂-P), 1.67 - 1.55 (m, 8H, P-O-CH₂-C*H*₂), 1.41 - 1.26 (m, 40H, C-C*H*₂), 0.95 - 0.85 (t, *J* = 6.5Hz, 12H, C-C*H*₃).

¹³C NMR (101 MHz, MeOD) δ 160.00, 134.57, 134.51, 134.45, 125.49, 115.95 (C*-Ar*), 68.46, 67.76, 67.73, 67.69, 51.32, 34.29, 33.01, 32.92, 31.67, 31.64, 31.61, 30.41, 30.29, 26.70, 23.75, 14.47 (C-*C*H₃).

³¹P NMR (162 MHz, MeOD) δ 27.11.

### 1.9. Step 9 - Preparation of a dendritic molecule of formula (I-A)

To a solution of 350.0 mg (0.434 mmol - 1.1 Eq.) of compound **(7)** in 15 mL of EtOAc were added 120 mg (0.1 Eq.) of Pd/C 10% as catalyst. The resulting mixture was purged with an atmosphere of hydrogen (five times), then vigorously stirred at RT for 5h. In a separate flask, 300 mg (0.398 mmol - 1 Eq.) of compound **(3)** were dissolved in 10.0 mL of CH₂Cl₂ before 0.22 mL (1.2 mmol - 3.0 Eq.) of oxalyl chloride (COCl)₂ and 4 drops of dimethyl formamide (DMF) were added. The orange solution was stirred at RT for 5h. When TLC confirmed the full consumption of compound **(7),** the catalyst was filtered over Celite and the crude product was concentrated under reduced pressure at RT. The acyl chloride was simultaneously concentrated under reduced pressure, dissolved in 10 mL of CH₂Cl₂ then the crude primary amine (intermediate product formed but not purified) and 0.16 mL (0.916 mmol - 2.3 Eq.) of N,N-Diisopropylethylamine (DIPEA) were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced pressure to yield 7.17 g orange oil. Purification by flash chromatography (100% EtOAc then CH₂Cl₂/EtOAc/MeOH 80/12/8 to 6/0/4) were conducted to separate the pure and impure fractions, to afford the dendritic molecule of formula **(I-A)** (487.2 mg, 81%) as a colorless oil.

### Analysis:

¹H NMR (400 MHz, MeOD) δ 7.23 (s, 2H), 6.85 (m Hz, 3H), 4.22 (m, 4H), 4.17 (t, *J* = 5.7 Hz, 2H), 3.97 (m, 8H), 3.91 - 3.84 (t, *J* = 4.6 Hz, 2H), 3.83 - 3.76 (m, 2H), 3.78 - 3.68 (m, 6H), 3.69 - 3.57 (m, 20H), 3.56 - 3.48 (m, 6H), 3.33 (m, 9H), 3.24 - 3.14 (ABx, *J* = 22 Hz, 4H), 1.60 (m, 8H), 1.29 (m, 38H), 0.90 (t, *J* = 6.8 Hz, 12H).

³¹P NMR (162 MHz, CDCl₃) δ 25.88.

### EXAMPLE 2: Synthesis of a dendritic molecule of formula (I-B) according to the invention

In this example, a dendritic molecule of the following formula (I-B) was prepared:

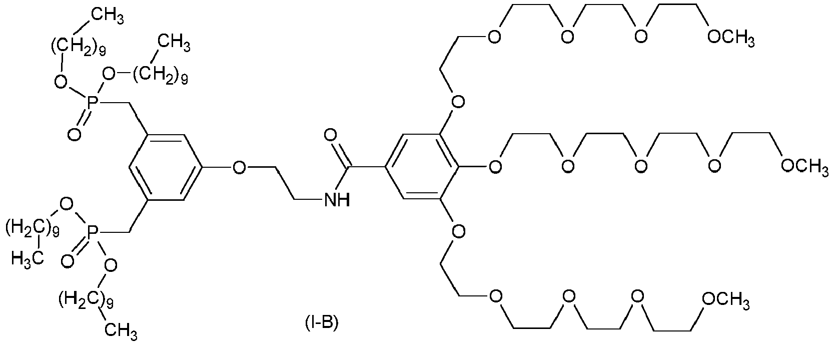

### 2.1. Step 1 - Preparation of compound (8)

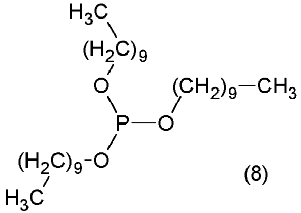

In a 100mL two-necked flask connected to a Dean-Stark, 91.4 mL (469.0 mmol - 6.0 Eq.) of decanol was added, then the reaction setup was stirred and passed under a flow of argon for 10 min.

After that, the liquid was heated to 75°C and the flow replaced by 2 balloons of argon. 9.51 mL (78.2 mmol - 1.0 Eq.) of trimethylphosphite were added slowly from the syringe and then the setup was heated at 220°C for 6 h.

Then the setup was cooled to ambient temperature, and the Dean-Stark containing the methanol formed during the reaction removed. An orange oil was obtained.

Distillation under reduced pressure was used to separate the remaining decanol from the compound (8) thus formed, at 160°C.

36.2 g of compound (8) as a colourless oil were recovered (yield = 92%).

### Analysis:

¹H NMR (400 MHz, CDCl₃) δ 3.77 (m, 6H), 1.64 - 1.55 (m, 6H), 1.41 - 1.18 (m, 44 H), 0.87 (t, *J* = 6.8 Hz, 9H).

¹³C NMR (101 MHz, CDCl₃) δ 62.48, 62.37, 32.04, 32.02, 31.33, 31.28, 29.76, 29.75, 29.71, 29.67, 29.64, 29.47, 29.43, 26.00, 22.82, 14.23.

31P-NMR (400MHz, CDCl₃): δ 139.19.

### 2.2. Step 2 - Preparation of compound (9)

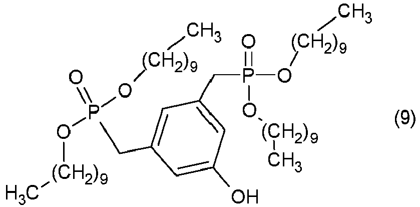

2.0 g (7.14 mmol - 1.0 Eq.) of compound **(4)** as prepared in step 1.4 of Example 1 above and 14.4 g (28.6 mmol - 4.0 Eq.) of compound **(8)** were added to a 100 mL flask. The reaction was stirred and heated to 140°C. overnight. A yellow-orange oil was obtained; a TLC was carried out (Petroleum ether / EtOAc 1/1).

The next day, after having cooled the solution, a Petroleum Ether / EtOAc chromatographic column was produced (1/1 to 2/3).

4.9 g of compound **(9)** were recovered (yield = 81 %).

### Analysis:

¹H NMR (400 MHz, CDCl₃) δ 6.81 - 6.77 (m, 2H), 6.67 (s, 1H), 3.92 (m, 8H), 3.11 - 3.01 (m, 4H), 1.67 - 1.51 (m, 8H), 1.26 (m, 56H), 0.94 - 0.82 (t, *J* = 6.9 Hz, 12H).

³¹P NMR (162 MHz, CDCl₃) δ 26.40

HRMS: m/z theo: C₄₈H₉₂O₇P₂ 842.63 g.mol⁻¹, measured: C₄₈H₉₂O₇P₂Na: 866.235 g.mol⁻¹

### 2.3. Step 3 - Preparation of compound (10)

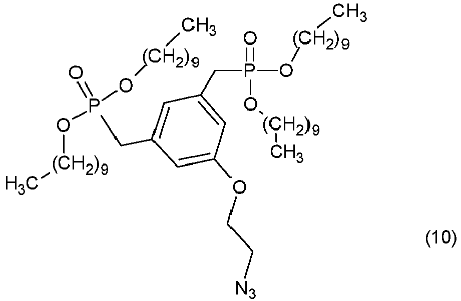

In a 100 mL flask were added 2.45 g (2.91 mmol - 1.0 Eq.) of compound **(9),** 0.25 g (4.36 mmol - 1.5 Eq.) of KOH and 49 g (0.3 mmol - 0.1 Eq.) of KI in 50 mL of toluene. The solution was stirred for 20 min and heated to 60°C. to dissolve the reagents. 1.05 g (4.36 mmol - 1.5 Eq.) of Linker 2 (as prepared above in step 1.7. of example 1) were added and the reaction was left to stir overnight.

The flask was cooled to room temperature, then the toluene was evaporated off under reduced pressure. The crude was then dissolved in DCM, and filtered through Celite. A chromatographic column was carried out DCM / MeOH (1/0 to 98/2 to 96/4). 2.1 g of compound **(10)** (79%) were thus recovered.

### Analysis:

¹H NMR (400 MHz, MeOD) δ 6.90 - 6.82 (m, 3H), 4.17 (t, *J* = 4.8 Hz, 2H), 3.98 (m, 8H), 3.63 - 3.56 (t, *J* = 4.9 Hz, 2H), 3.25 - 3.13 (d, *J* = 22 Hz, 4H), 1.62 (p, *J* = 6.6 Hz, 8H), 1.32 (m, 54H), 0.96 - 0.85 (t, *J* = 6.5 Hz, 12H).

¹³C NMR (101 MHz, MeOD) δ 160.00, 134.57, 134.45, 125.47, 115.96, 68.46, 67.77, 67.73, 67.70, 51.32, 34.31, 33.12, 32.94, 31.67, 31.64, 31.61, 30.75, 30.74, 30.51, 30.33, 26.71, 23.78, 14.48.

³¹P NMR (162 MHz, MeOD) δ 31.03.

### 2.4. Step 4 - Preparation of a dendritic molecule of formula (I-B)

To a solution of 237 mg (0.259 mmol - 1.1 Eq.) of compound **(10)** in 15 mL of EtOAc and 251 mg (0.1 Eq.) of Pd/C 10% as catalyst. The resulting mixture was purged with an atmosphere of hydrogen (five times), then vigorously stirred at RT for 5h. In a separate flask, 175 mg (0.236 mmol - 1.0 Eq.) of compound **(3)** as prepared above at step 1.3. of example 1 were dissolved in 15 mL of CH₂Cl₂ before 0.13 mL (0.71 mmol - 3.0 Eq.) of (COCl)₂ and 4 drops of DMF were added. The orange solution was stirred at RT for 5h. When TLC confirmed the full consumption of compound **(3),** the catalyst was filtered over Celite and the crude product was concentrated under reduced pressure at RT. The acyl chloride was simultaneously concentrated under reduced pressure, dissolved in CH₂Cl₂ then the crude primary amine and 0.11 mL (0.916 mmol - 2.3 Eq.) of DIPEA were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced pressure to yield to an orange oil. Purification by flash chromatography (100% EtOAc then CH₂Cl₂/EtOAc/MeOH 80/12/8 to 6/0/4) were conducted to separate the pure and impure fractions, to afford the dendritic molecule of formula **(I-B)** (293 mg, 67%) as colorless oil.

### Analysis:

¹H NMR (400 MHz, MeOD) δ 7.23 (s, 2H), 6.85 (m, 2H), 4.26 - 4.13 (m, 6H), 4.08 - 3.90 (m, 8H), 3.88 - 3.85 (t, *J* = 4.2 Hz, 2H), 3.81 - 3.78 (m, 1H), 3.76 (t, *J* = 5.7 Hz, 1H), 3.71 (m, 4H), 3.67 - 3.57 (m, 19H), 3.55 - 3.49 (m, 4H), 3.37 - 3.31 (bs, 9H), 3.24 - 3.14 (ABₓ, *J* = 21.9 Hz, 4H), 1.67 - 1.55 (m, 8H), 1.29 (m, 55H), 0.90 (t, *J* = 6.9Hz, 12H).

³¹P NMR (162 MHz, MeOD) δ 27.14.

### EXAMPLE 3: Synthesis of a dendritic molecule of formula (I-C) according to the invention

In this example, a dendritic molecule of the following formula **(I-C)** was prepared:

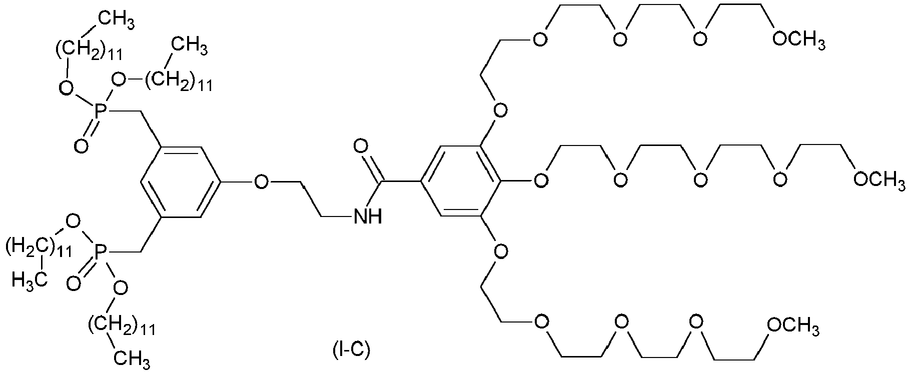

### 3.1. Step 1 - Preparation of compound (11)

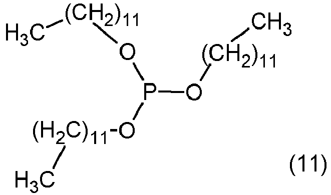

In a 250 mL two-necked flask connected to a Dean-Stark, 107.0 mL (469.0 mmol - 6.0 Eq.) of dodecanol was added, then the reaction setup was stirred and passed under a flow of argon for 10 min.

After that, the liquid was heated to 75°C and the flow replaced by 2 balloons of argon. 9.51 mL (78.2 mmol - 1.0 Eq.) of trimethylphosphite were added slowly from the syringe and then the setup was heated at 220°C for 6 h.

Then the setup was cooled to ambient temperature, and the Dean-Stark containing the methanol formed during the reaction removed. An orange oil was obtained.

Distillation under reduced pressure was used to separate the remaining dodecanol from the compound **(11)** thus formed, at 160°C.

43.0 g of compound **(11)** as a colourless oil were recovered (yield = 94%).

### Analysis:

¹H NMR (400 MHz, CDCl₃) δ 3.78 (m, 6H), 1.64 - 1.51 (m, 6H), 1.26 (m, 65H), 0.91 - 0.83 (t, *J* = 6.5 Hz, 9H).

¹³C NMR (101 MHz, CDCl₃) δ 62.97, 62.94, 62.44, 62.33, 32.88, 32.03, 31.29, 31.24, 29.79, 29.76, 29.73, 29.72, 29.58, 29.47, 29.44, 25.96, 25.89, 22.79, 14.17.

³¹P NMR (162 MHz, CDCl₃) δ 139.19.

### 3.2. Step 2 - Preparation of compound (12)

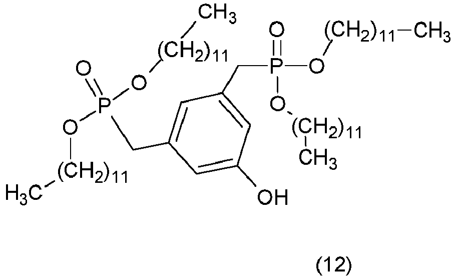

1.0 g (3.57 mmol - 1.0 Eq.) of compound **(4)** as prepared in step 1.4 of Example 1 above and 8.39 g (14.3 mmol - 4.0 Eq.) of compound **(11)** were added to a 100 mL flask. The reaction was stirred and heated to 140°C. overnight. A yellow-orange oil was obtained; a TLC was carried out (Petroleum ether / EtOAc 1/1).

The next day, after having cooled the solution, a Petroleum Ether / EtOAc chromatographic column was produced (1/1 to 2/3).

2.28 g of compound **(12)** were recovered (yield = 67 %).

### Analysis:

¹H NMR (400 MHz, CDCl₃) δ 6.80 (s, 2H), 6.66 (s, 1H), 3.92 (dq, *J* = 13.0, 8.0 Hz, 8H), 3.11 - 3.01 (m, 4H), 1.58 (t, *J* = 6.6 Hz, 8H), 1.25 (s, 75H), 0.88 (t, *J* = 6.9 Hz, 12H).

¹³C NMR (101 MHz, CDCl₃) δ 157.61, 132.68, 122.76, 115.79 (*C,* Ar), 66.45 (*C*H₂*-*O*-*R)*,* 31.94, 30.61, 29.70, 29.68, 29.64, 29.59, 29.38, 29.25, 25.53, 23.84, 22.70 (*C*H₂), 14.12 (*C*H₃).

³¹P NMR (162 MHz, CDCl₃) δ 26.40.

C₄₈H₉₂NaO₇P₂ m/z calculated: 978,3898, m/Z found: 978,3990.

### 3.3. Step 3 - Preparation of compound (13)

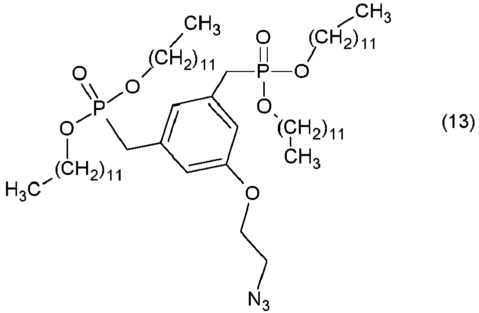

In a 100 mL flask were added 500 mg (0.523 mmol - 1.0 Eq.) of compound **(12),** 44.0 mg (0.785 mmol - 1.5 Eq.) of KOH and 26 mg (0.157 mmol - 0.1 Eq.) of KI in 25 mL of toluene. The solution was stirred for 20 min and heated to 60°C. to dissolve the reagents. 252.4 mg (0.785 mmol - 1.5 Eq.) of Linker 2 (as prepared above in step 1.7. of example 1) were added and the reaction was left to stir overnight.

The flask was cooled to room temperature, then the toluene was evaporated off under reduced pressure. The crude was then dissolved in DCM, and filtered through Celite. A chromatographic column was carried out DCM / MeOH (1/0 to 98/2 to 96/4). 500 mg of compound **(13)** (93%) were thus recovered.

### Analysis:

¹H NMR (400 MHz, CDCl₃) δ 6.87 - 6.68 (m, 3H), 4.13 (t, *J* = 5.0 Hz, 2H), 4.01 - 3.85 (m, 8H), 3.56 (d, *J* = 5.1 Hz, 2H), 3.08 (ABx, *J* = 21.9 Hz, 4H), 1.64 - 1.52 (m, 8H), 1.32 - 1.20 (m, 72H), 0.88 (t, *J* = 6.6 Hz, 12H).

³¹P NMR (162 MHz, CDCl₃) δ 25.94.

### 3.4. Step 4 - Preparation of a dendritic molecule of formula (I-C)

To a solution of 305 mg (0.297 mmol - 1.1 Eq.) of compound **(13)** in 15 mL of EtOAc were added 320 mg (0.1 Eq.) of Pd/C 10% as catalyst. The resulting mixture was purged with an atmosphere of hydrogen (five times), then vigorously stirred at RT for 5h. In a separate flask, 200 mg (0.270 mmol - 1.0 Eq.) of compound **(3)** as prepared above at step 1.3. of example 1 were dissolved in 15 mL of CH₂Cl₂ before 0.15 mL (0.81 mmol - 3.0 Eq.) of (COCl)₂ and 4 drops of DMF were added. The orange solution was stirred at RT for 5h. When TLC confirmed the full consumption of compound **(3),** the catalyst was filtered over Celite and the crude product was concentrated under reduced pressure at RT. The acyl chloride was simultaneously concentrated under reduced pressure, dissolved in CH₂Cl₂ then the crude primary amine and 0.11 mL (0.916 mmol - 2.3 Eq.) of DIPEA were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced Purification by flash chromatography (100% EtOAc then CH₂Cl₂/EtOAc/MeOH 80/12/8 to 6/0/4) were conducted to separate the pure and impure fractions, to afford the dendritic molecule of formula **(I-C)** (265 mg, 58%) as colorless oil.

### Analysis:

¹H NMR (400 MHz, CDCl₃) δ 7.12 (s, 2H), 6.74 (m, 3H), 4.19 (m, 4H), 4.09 (t, *J* = 5.8 Hz, 2H), 3.90 (m, 8H), 3.82 (t, *J* = 4.8 Hz, 2H), 3.78 - 3.71 (m, 4H), 3.61 (m, 19H), 3.51 - 3.48 (m, 4H), 3.35 - 3.27 (m, 9H), 3.10 - 2.97 (ABx, *J* = 21.8 Hz, 4H), 1.55 (h, *J* = 6.2 Hz, 8H), 1.22 (m, 76H), 0.84 (t, *J* = 6.4 Hz, 12H).

³¹P NMR (162 MHz, CDCl₃) δ 25.88.

### EXAMPLE 4: Synthesis of a dendritic molecule of formula (I-D) according to the invention

In this example, a dendritic molecule of the following formula **(I-D)** was prepared:

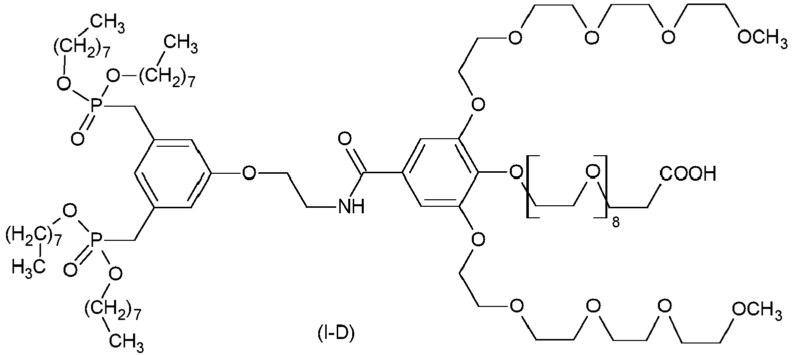

### 4.1. Step 1 - Preparation of compound (14)

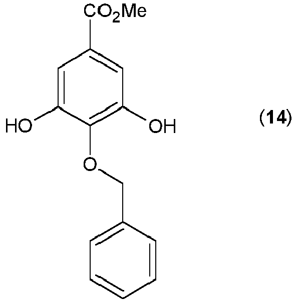

To a solution of 30 g (163 mmol - 1 Eq.) of methyl gallate in 150 mL of DMF were successively added 49.1 g (489 mmol - 4.5 Eq.) of KHCO₃, 0.136 g (0.82 mmol - 0.006 Eq.) of KI and 21.3 mL (163 mmol - 1.0 Eq.) of benzyl bromide (BnBr). The resulting mixture was stirred at RT for 36h.

The solids were filtered over Celite, the filtrate was acidified with an aqueous solution of HCl 2N (50.0 mL). The aqueous mixture was extracted with EtOAc (three times), the combined organic layer was washed with an aqueous saturated solution of NaHCO₃ (two times), brine (three times), dried over Na₂SO₄, filtered, concentrated under reduced pressure and dried under vacuum overnight. Purification with a chromatographic column (DCM/MeOH, 100:0 to 98:2 to 96:4 to 90:10) gave a translucent oil. The mixture was then dissolved with a small amount of AcOEt and Petroleum ether was poured slowly until precipitation occurred. Compound **(14)** was obtained as a white solid (16.5g, 38%)

### Analysis:

¹H NMR (400 MHz, MeOD) δ 7.53 - 7.44 (m, 2H, Ar-*H*), 7.30 (qd, *J* = 6.9, 3.7 Hz, 3H, Ar*-H*), 7.01 (d, *J* = 1.3 Hz, 2H, Ar-*H*), 5.14 (s, 2H, Ar-C*H*₂), 3.82 (d, *J* = 1.5 Hz, 3H, O-C*H*₃).

¹³C NMR (101 MHz, MeOD) δ 168.49, 151.90, 139.62, 138.80, 129.87, 129.19, 129.16, 129.11, 126.44 (12C, C-Ar), 110.07 (1C, C-COOR), 75.12 (1C, Ar-CH₂), 52.46 (1C, O-CH₃).

### 4.2. Step 2 - Preparation of compound (15)

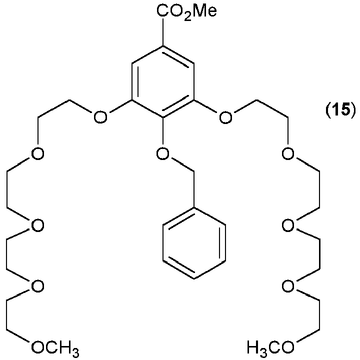

To a solution of 5.81 g (16.1 mmol - 2.2 Eq.) of compound **(14)** in 150.0 mL of acetone were added 3.22 g (23.4 mmol - 3.2 Eq.) of K₂CO₃,0.12 g (0.73 mmol - 0.4 Eq.) of KI and 2.0 g (7.3 mmol - 1.0 Eq.) of compound **(1)** as prepared in step 1.1 of example 1. The resulting solution was heated to reflux overnight.

The reaction mixture was cooled to RT, the solvent was removed, solids were suspended in CH₂Cl₂, filtered over Celite, washed with an aqueous solution of Na₂S₂O₃ 2N and brine, dried over Na₂SO₄, filtered and concentrated under reduced pressure. Purification by flash chromatography (CH₂Cl₂/MeOH 98/2 to 96/4 to 9/1) gave 3.3 g (71%) of compound **(15)** in the form of a yellowish oil.

### Analysis:

¹H NMR (400 MHz, Chloroform-d) δ 7.46 (d, 2H : Ar-*H*), 7.35 - 7.20 (m, 5H : Ar-*H*), 5.09 (s, 2H : -C*H*₂-OAr), 4.15 (dd, *J* = 5.6, 4.2 Hz, 4H: -O-C*H*₂-R), 3.86 (s, 3H : C*H*₃-O-C=O-R), 3.85 - 3.81 (m, 4H : -O-CH₂-C*H*₂-R), 3.70 - 3.66 (m, 4H : -O-C*H*₂-R), 3.63 - 3.56 (m, 16H : -O-C*H*₂-R), 3.51 - 3.47 (m, 4H : -O-C*H*₂-R), 3.33 (s, 6H : C*H₃*-O-).

¹³C NMR (101 MHz, CDCl₃) δ 169.26, 153.95, 142.97, 139.17, 129.74, 129.22, 129.01, 127.17, 109.67, 75.95, 72.93, 71.83, 71.65, 71.57, 71.50, 71.32, 70.81, 69.98, 59.06.

### 4.3. Step 3 - Preparation of compound (16)

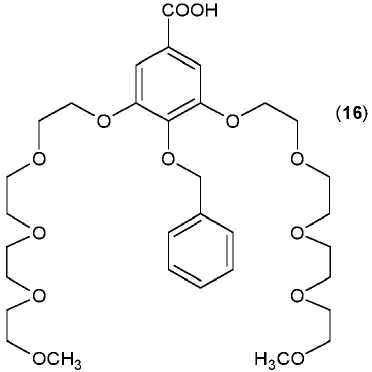

1.01 g (25.22 mmol - 5.0 Eq.) of NaOH were added to a solution of 3.3 g (5.04 mmol - 1.0 Eq.) of compound **(15)** in 27 mL of MeOH and 3 mL of distilled water. The orange solution was stirred at RT overnight.

The solvent was removed, the crude product was suspended in CH₂Cl₂, quenched with an aqueous solution of HCl 2N, the aqueous layer was extracted with CH₂Cl₂ (five times), the combined organic layer was dried over Na₂SO₄, filtered and concentrated under reduced pressure to yield to compound **(16)** (3.05 g, 95%) as a light yellow oil.

### Analysis:

¹H NMR (400 MHz, Chloroform-d) δ 7.46 (d, 2H : Ar-*H*), 7.35 - 7.20 (m, 5H : Ar-*H*), 5.09 (s, 2H : -C*H*₂-OAr), 4.15 (dd, *J* = 5.6, 4.2 Hz, 4H: -O-C*H*₂-R), 3.85 - 3.81 (m, 4H : -O-CH₂-C*H*₂-R), 3.70 - 3.66 (m, 4H : -O-C*H₂*-R), 3.63 - 3.56 (m, 16H : -O-C*H₂*-R), 3.51 - 3.47 (m, 4H : -O-C*H₂*-R), 3.33 (s, 6H : *CH3-O-).*

### 4.4. Step 4 - Preparation of compound (17)

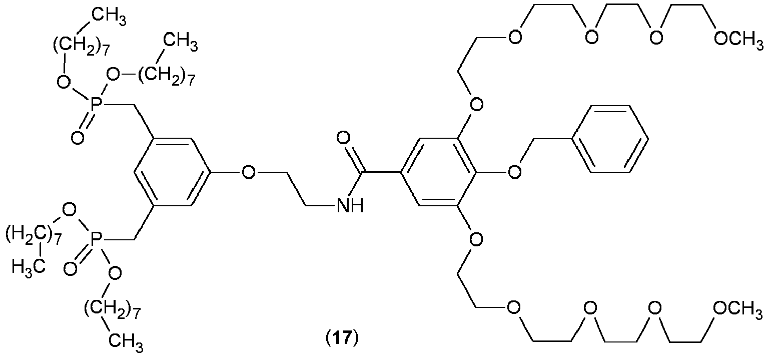

To a solution of 1.67 g (2.09 mmol - 1.0 Eq.) of compound **(7)** as prepared at step 1.8 of example 1 in 40 mL of EtOAc were added 1.1 g (0.1 Eq.) of Pd/C 10% as catalyst. The resulting mixture was purged with an atmosphere of hydrogen (five times), then vigorously stirred at RT for 5h. When TLC confirmed the full consumption of compound **(7),** the catalyst was filtered over Celite and the crude product was concentrated under reduced pressure at RT. In parallel, compound **(16)** (1.00 eq., 2.09 mmol, 1.34 g) was dissolved in DCM (67.0 mL), then 1-ethyl-3-(3-dimethylaminopropyl)carbodiimide (EDC) (1.50 eq., 3.13 mmol, 612 mg), and DMAP (0.10 eq., 0.21 mmol, 26 mg) were added, and the reaction stirred for 10 min before adding the crude primary amine. The resulting mixture was stirred at RT overnight. Monitoring by TLC DCM/MeOH, 6:4, KMnO4. The reaction mixture was concentrated under reduced pressure, the crude was purified by flash chromatography (SiO₂ 40.0 g, solid deposit in DCM, eluent: DCM/MeOH 100/0 to 90/10) and give expected product **(17)** (2.55 g, 89% yield) as a colorless oil.

### Analysis:

1H NMR (400 MHz, MeOD) :δ 7.54 - 7.52 (m, 2H, Ar-*H*), 7.36 - 7.28 (m, 3H, Ar-*H*), 7.21 (s, 2H, Ar-*H*), 6.86 - 6.85 (m, 3H, Ar-*H*), 5.49 (s, 3H, ?), 5.10 (s, 2H, O-C*H*₂-Ar), 4.19 (t, 4H, Ar-O-C*H*₂-CH₂-O, *J*), 4.18 (t, 2H, O-C*H*₂-CH₂-N, *J),* 3.99 - 3.94 (m, 9H, P-O-C*H*₂-CH₂), 3.88 - 3.86 (m, 4H, Ar-O-CH₂-C*H*₂-O), 3.77 - 3.74 (t, 2H, O-CH₂-C*H*₂-N, J), 3.72 - 3.69 (m, 4H, O-CH₂-C*H*₂-O), 3.63 - 3.54 (m, 17H, O-CH₂-C*H*₂-O), 3.49 - 3.47 (m, 4H, ...), 3.31 (s, 6H, O-C*H*₃), 3.19 (d, 4H, P-C*H*₂, J), 1.62 - 1.58 (m, 9H, P-O-CH₂-C*H*₂-), 1.33 - 1.28 (m, 44H), 0.89 (t, 12H, CH₂-C*H*₃, *J).*

¹³C NMR (101 MHz, MeOD) δ 169.56, 160.38, 154.04, 141.70, 139.20, 130.67, 129.73, 129.22, 129.00, 115.98, 107.49, 75.94, 72.93, 71.83, 71.64, 71.56, 71.51, 71.32, 70.82, 69.99, 67.74, 67.71, 67.67, 67.54, 59.09, 54.80, 34.29, 32.98, 31.65, 31.62, 31.59, 30.69, 30.38, 30.26, 26.68, 23.73, 14.49.

³¹P NMR (400 MHz, Methano-d4): δ 27.16

### 4.5. Step 5 - Preparation of compound (18)

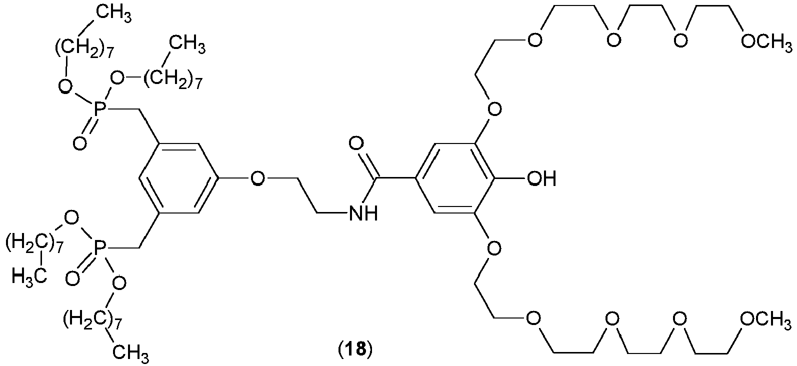

To a solution of 2.55g (1.83 mmol - 1.0 Eq.) of compound **(17)** in 40.0 mL of EtOAc were added 355.0 mg of Pd/C 10% as catalyst. The heterogeneous solution was purged with an atmosphere of hydrogen (five times), then vigorously stirred at RT overnight, the catalyst was filtered off over Celite, the crude product was concentrated under reduced pressure, to give 2.32compound **(18)** as a colorless oil (97%).

### Analysis:

¹H NMR (400 MHz, MeOD) δ 7.24 (s, 2H), 6.85 (m, 3H), 4.25 - 4.18 (m, 4H), 4.16 (t, *J* = 5.7 Hz, 2H), 4.13 - 4.05 (m, 1H), 4.06 - 3.90 (m, 8H), 3.91 - 3.84 (m, 4H), 3.78 - 3.69 (m, 6H), 3.68 - 3.58 (m, 19H), 3.55 - 3.47 (m, 4H), 3.33 (s, 6H), 3.24 - 3.13 (ABx, *J* = 21.9 Hz, 4H), 1.59 (m, 8H), 1.37 - 1.20 (m, 44H), 0.90 (t, *J* = 6.85 Hz, 12H).

¹³C NMR (101 MHz, MeOD) δ 169.79, 148.23, 141.70, 125.33, 115.98, 108.36, 72.96, 71.66, 71.58, 71.53, 71.36, 70.77, 70.03, 67.74, 67.67, 61.53, 59.09, 58.32, 40.71, 34.28, 33.00, 31.66, 31.60, 30.67, 30.40, 30.27, 26.69, 23.74, 20.86, 18.37, 14.49, 14.46.

³¹P NMR (162 MHz, MeOD) δ 27.17.

### 4.6. Step 6 - Preparation of Linker 3 (L3)

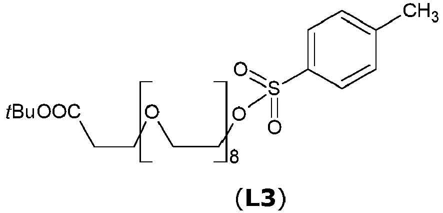

To 3.00 g (6.02 mmol - 1 Eq.) of t-Butyl 1-hydroxy-3,6,9,12,15,18,21,24-octaoxaheptacosan-27-oate in 50 mL of DCM, was added 1.69 mL (12.04 mmol - 2 Eq.) of Et₃N slowly, the mixture was stirred 10 min, then 2.32 g (12.04 mmol - 2 Eq.) de tosyl chloride was added slowly.

After the night TLC (EtP, AcOEt 7:3, KMnO₄) showed no traces of starting material. The mixture was concentrated, tosyl chloride salts were precipitated in AcOEt and filtered over celite pad, washed 3 times with AcOEt, to give the crude product (orange oil). It was then purified by Flash chromatography, Interchim-80g-50, liquid deposit in EtP/AcOEt (9:1), Eluent: 15 min EtP/AcOEt (9:1), 10 min EtP/AcOEt (8:2) with isocratic, and 10min EtP/AcOEt (7:3).

3.52 g of Linker 3 as a colourless oil was recovered (90%)

### Analysis:

¹H NMR (400 MHz, MeOD) δ 7.80 (d, *J* = 8.3 Hz, 2H), 7.45 (d, *J* = 8.2 Hz, 2H), 4.14 (t, *J* = 4.5 Hz, 2H), 3.77 - 3.50 (m, 32H), 2.51 - 2.43 (m, 5H), 1.45 (s, 9H).

### 4.7. Step 7 - Preparation of compound (19)

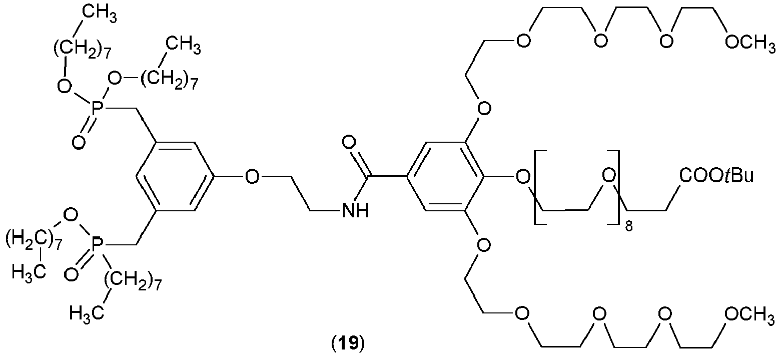

To a solution of 1.0 g (0.76 mmol - 1.0 Eq) of compound **(18)** in 25 mL of acetone, 0.48 g (0.84 mmol - 1.1 Eq.) of Linker 3, 51.3 mg (0.30 mmol - 0.4 Eq.) of KI and 340 mg (2.45 mmol - 3.2 Eq.) of K₂CO₃ were successively added. The resulting reaction mixture was heated to reflux overnight.

The reaction mixture was cooled to RT, the solvent was removed, the crude product was suspended in CH₂Cl₂, the solids were filtered off; the crude product was concentrated under reduced pressure. Purification by flash chromatography (CH₂Cl₂/MeOH 95/5 to 8/2 to 6/4) afforded 827 mg of pure compound **(19)** as a yellowish oil (65% yield).

### Analysis:

¹H NMR (400 MHz, MeOD) δ 7.26 (s, 2H), 6.86 (m, 3H), 4.29 - 4.22 (m, 6H), 4.18 (t, *J* = 5.6 Hz, 2H), 3.97 (m, 8H), 3.91 - 3.86 (m, 4H), 3.78 (m, 3H), 3.73 - 3.50 (m, 48H), 3.34 (s, 6H), 3.25 - 3.11 (ABx, *J* = 21.9 Hz, 4H), 2.48 (t, *J* = 6.2 Hz, 2H), 1.67 - 1.55 (m, 8H), 1.41 - 1.23 (m, 44H), 0.94 - 0.86 (t, *J* = 6.7 Hz 12H).

¹³C NMR (101 MHz, MeOD) δ 172.76, 169.27, 153.62, 140.98, 134.50, 131.15, 116.01, 107.25, 81.74, 73.60, 72.94, 71.51, 71.44, 71.29, 71.25, 71.23, 71.15, 71.10, 71.05, 70.99, 70.51, 69.57, 67.85, 67.75, 67.68, 67.51, 59.16, 58.32, 40.80, 37.18, 34.27, 32.99, 31.67, 31.61, 30.40, 30.27, 28.41, 26.69, 23.74, 18.37, 14.49.

³¹P NMR (162 MHz, MeOD) δ 27.17.

### 4.8. Step 8 - Preparation of a dendritic molecule of formula (I-D)

In a flask containing 826 mg (0.48 mmol - 1.0 Eq.) dissolved in 8 mL of DCM, 1.23 mL (15.9 mmol, 33 Eq). of trifluoroacetic acid (TFA) was added dropwise. The mixture was stirred for 2h, then evaporated under reduced pressure, affording the dendritic molecule of formula **(I-D)** as a colorless oil (91%).

### Analysis:

¹H NMR (400 MHz, MeOD) δ 7.25 (s, 2H), 6.86 (m, 3H), 4.28 - 4.20 (m, 6H), 4.17 (t, *J* = 5.6 Hz, 2H), 3.97 (m, 8H), 3.92 - 3.85 (m, 4H), 3.82 - 3.73 (m, 4H), 3.76 - 3.53 (m, 45H), 3.55 - 3.48 (m, 4H), 3.33 (s, 6H), 3.20 (ABx, *J* = 21.9 Hz, 4H), 2.54 (t, *J* = 6.2 Hz, 2H), 1.67 - 1.53 (m, 8H), 1.31 (m, 44H), 0.90 (t, *J* = 7.0 Hz, 11H).

¹³C NMR (101 MHz, MeOD) δ 167.99, 152.31, 129.42, 114.57, 106.20, 72.22, 71.56, 70.20, 70.10, 70.00, 69.92, 69.88, 69.85, 69.26, 68.43, 66.38, 66.35, 66.28, 66.13, 57.73, 39.41, 34.37, 32.87, 31.59, 30.26, 30.20, 28.99, 28.87, 25.29, 22.33, 13.08.

³¹P NMR (162 MHz, MeOD) δ 27.17.

### EXAMPLE 5: Synthesis of a dendritic molecule of formula (I-E) according to the invention

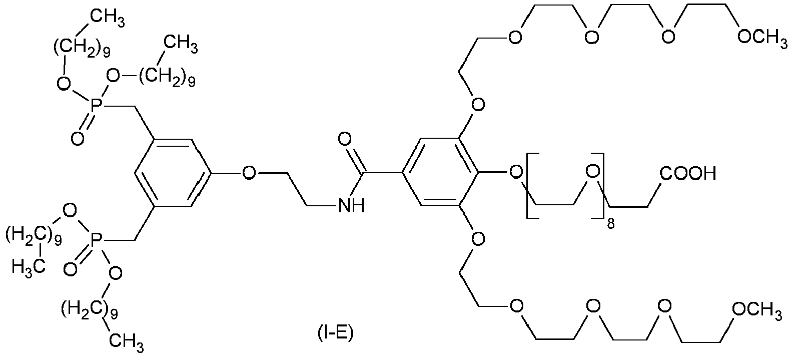

### 5.1. Step 1 - Preparation of compound (20)

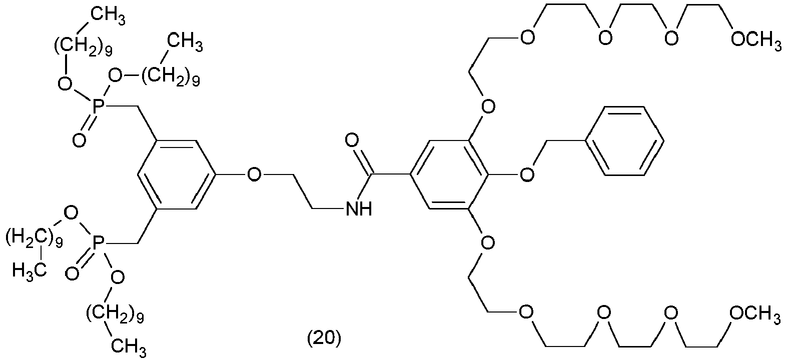

To a solution of 1.64 g (1.80 mmol - 1.0 Eq.) of compound **(10)** as prepared at step 3 of example 4 in 40 mL of EtOAc were added 960 mg (0.1 Eq.) of Pd/C 10% as catalyst. The resulting mixture was purged with an atmosphere of hydrogen (five times), then vigorously stirred at RT for 5 h.

When TLC confirmed the full consumption of compound **(10),** the catalyst was filtered over Celite and the crude product was concentrated under reduced pressure at RT. In parallel, compound **(16)** (1.1 eq, 1.98 mmol, 1.27 g) was dissolved in DCM (60.0 mL), then EDC (1.50 eq, 2.7 mmol, 529 mg), and DMAP (0.10 eq, 0.18 mmol, 22 mg) were added, and the reaction stirred for 10 min before adding the crude primary amine. The resulting mixture was stirred at RT overnight. Monitoring by TLC DCM/MeOH, 6:4, KMnO4. The reaction mixture was concentrated under reduced pressure, the crude was purified by flash chromatography

(SiO₂ 40.0 g, solid deposit in DCM, eluent: DCM/MeOH 100/0 to 90/10) and give expected product **(20)** (2.41 g, 88% yield) as a colorless oil

### Analysis:

¹H NMR (400 MHz, MeOD) δ 7.46 - 7.39 (s, 2H), 7.28 - 7.14 (m, 3H), 7.11 (s, 2H), 6.79 - 6.72 (m, 3H), 5.00 (s, 2H), 4.13 - 4.03 (m, 6H), 3.91 - 3.81 (m, 8H), 3.79 - 3.75 (m, 4H), 3.65 (t, *J* = 5.6 Hz, 2H), 3.62 - 3.59 (m, 4H), 3.55 - 3.42 (m, 17H), 3.40 - 3.36 (m, 4H), 3.21 (s, 6H), 3.09 (ABx, *J* = 21.4 Hz, 4H), 1.56 - 1.44 (m, 8H), 1.31 - 1.11 (m, 56H), 0.79 (t, *J* = 6.7 Hz, 12H).

¹³C NMR (101 MHz, MeOD) δ 169.55, 160.40, 154.06, 141.75, 139.23, 130.67, 129.73, 129.22, 128.99, 116.01, 107.50, 75.94, 72.95, 71.85, 71.67, 71.58, 71.53, 71.35, 70.84, 70.00, 67.75, 67.68, 67.55, 59.09, 54.80, 40.78, 34.31, 33.11, 32.94, 31.67, 31.61, 30.75, 30.73, 30.67, 30.52, 30.32, 26.70, 23.78, 14.50.

³¹P NMR (162 MHz, MeOD) δ 27.15.

### 5.2. Step 2 - Preparation of compound (21)

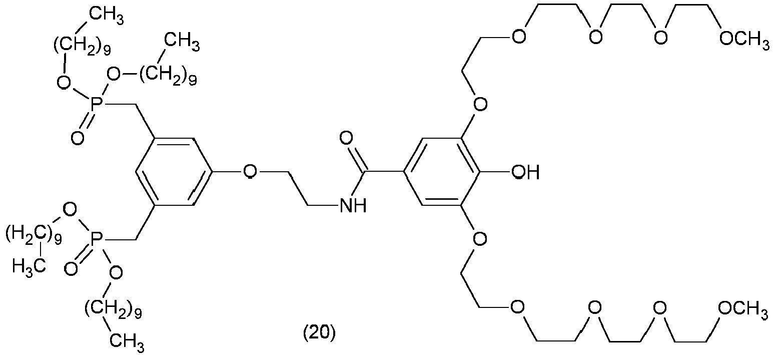

To a solution of 2.41 g (1.6 mmol - 1.0 Eq.) of compound **(20)** in 40 mL of EtOAc were added 340.0 mg of Pd/C 10% as catalyst. The heterogeneous solution was purged with an atmosphere of hydrogen (five times), then vigorously stirred at RT for 5h, the catalyst was filtered off over Celite, the crude product was concentrated under reduced pressure, to give 2.25 g of compound **(21)** as a colorless oil (99%).

### Analysis:

¹H NMR (400 MHz, MeOD) δ 7.24 (s, 2H), 6.85 (m, 3H), 4.22 - 4.20 (m, 4H), 4.16 (t, J = 5.7 Hz, 2H), 4.13 - 4.07 (m, 2H), 4.01 - 3.92 (m, 8H), 3.89 - 3.85 (m, 3H), 3.77 - 3.71 (m, 6H), 3.69 - 3.57 (m, 26H), 3.54 - 3.47 (m, 4H), 3.33 (s, 6H), 3.19 (ABx, J = 22.0 Hz, 4H), 1.66 - 1.55 (m, 8H), 1.29 (m, 56H), 0.90 (d, J = 7.3 Hz, 12H).

¹³C NMR (101 MHz, MeOD): δ 172.97, 169.77, 148.23, 141.71, 125.32, 115.99, 108.37, 72.97, 71.67, 71.58, 71.54, 71.36, 70.77, 70.03, 67.75, 67.67, 61.53, 59.10, 58.32, 40.71, 34.30, 33.12, 32.94, 31.67, 31.64, 31.61, 30.75, 30.73, 30.67, 30.52, 30.32, 26.70, 23.78, 20.86, 18.37, 14.50, 14.47.

³¹P NMR (162 MHz, MeOD) δ 27.16.

### 5.3. Step 3 - Preparation of compound (22)

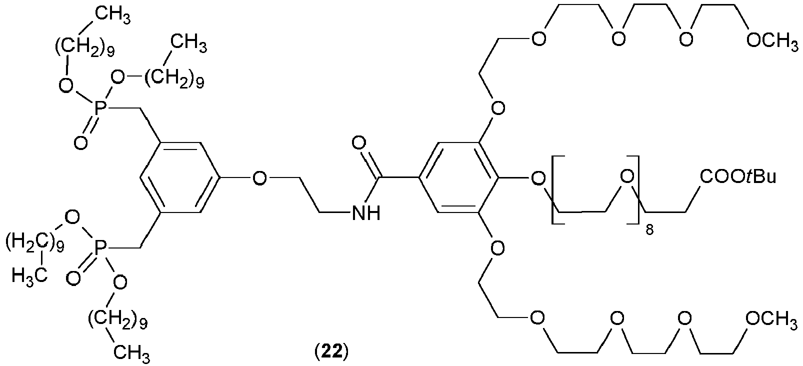

To a solution of 1.01 g (0.705 mmol - 1.0 Eq.) of compound **(21)** in 25 mL of acetone, 0.440 g (0.775 mmol - 1.1 Eq.) of Linker 3 as prepared at step 6 of example 48 mg (0.282 mmol - 0.4 Eq.) of KI and 313 mg (2.26 mmol - 3.2 Eq.) of K₂CO₃ were successively added. The resulting reaction mixture was heated to reflux overnight.

The reaction mixture was cooled to RT, the solvent was removed, the crude product was suspended in CH₂Cl₂, the solids were filtered off; the crude product was concentrated under reduced pressure. Purification by flash chromatography (CH₂Cl₂/MeOH 95/5 to 8/2 to 6/4) afforded 736 mg of compound **(22)** as a yellowish oil (59% yield).

### Analysis:

¹H NMR (400 MHz, MeOD) δ 7.27 (s, 2H), 6.86 (m, 3H), 4.30 - 4.22 (m, 6H), 4.18 (t, J = 5.6 Hz, 2H), 4.03 - 3.91 (m, 8H), 3.93 - 3.86 (m, 4H), 3.81 - 3.75 (m, 3H), 3.76 - 3.46 (m, 48H), 3.34 (s, 6H), 3.20 (ABx, J = 21.9 Hz, 4H), 2.48 (t, J = 6.2 Hz, 2H), 1.60 (m, 8H), 1.45 (s, 9H), 1.29 (s, 56H), 0.90 (t, *J* = 6.7 Hz, 12H).

¹³C NMR (101 MHz, MeOD) δ 153.63, 116.02, 107.32, 81.74, 72.95, 71.52, 71.46, 71.30, 70.53, 69.60, 67.86, 67.75, 59.15, 58.32, 37.19, 33.11, 31.67, 31.61, 30.75, 30.73, 30.51, 30.32, 28.40, 26.70, 23.77, 14.49.

³¹P NMR (162 MHz, MeOD) δ 27.16.

### 5.4. Step 4 - Preparation of a dendritic molecule of formula (I-E)

In a flask containing 736 mg (0.406 mmol - 1.0 Eq.) of compound **(22)** dissolved in DCM, 30 Eq. of TFA was added dropwise. The mixture was stirred for 2h, then evaporated under reduced pressure, affording 645 mg of the dendritic molecule of formula **(I-E)** as a colorless oil (93%)

### Analysis:

¹H NMR (400 MHz, MeOD) δ 7.15 (s, 2H), 6.76 (m, 3H), 4.18 - 4.11 (m, 6H), 4.07 (t, J = 5.6 Hz, 2H), 3.94 - 3.82 (m, 8H), 3.81 - 3.77 (m, 4H), 3.71 - 3.45 (m, 48H), 3.44 - 3.40 (m, 4H), 3.23 (s, 6H), 3.10 (ABx, J = 21.8 Hz, 4H), 2.48 (t, J = 6.2 Hz, 2H), 1.50 (q, J = 6.7 Hz, 8H), 1.30 - 1.14 (m, 56H), 0.84 - 0.73 (m, 12H).

### EXAMPLE 6: Synthesis of a dendritic molecule of formula (I-F) according to the invention

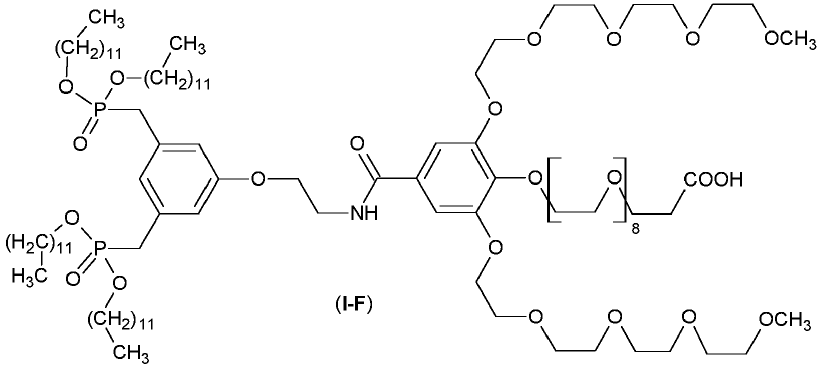

### 6.1. Step 1 - Preparation of compound (23)

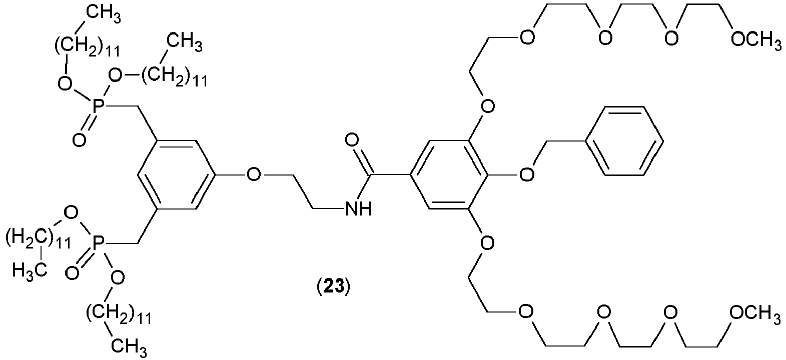

To a solution of 300 mg (0.312 mmol - 1.1 Eq.) of compound **(13),** as prepared at step 3 of example 3, in 15 mL of EtOAc were added 120 mg (0.1 Eq.) of Pd/C 10% as catalyst. The resulting mixture was purged with an atmosphere of hydrogen (five times), then vigorously stirred at RT for 5h. In a separate flask, 200 mg (0.260 mmol - 1.0 Eq.) of compound **(16),** as prepared in step 3 of example 4, were dissolved in 10 mL of CH₂Cl₂ before 0.1 mL (1.3 mmol - 3.0 Eq.) of (COCl)₂ and 4 drops of DMF were added. The orange solution was stirred at RT for 5h. When TLC confirmed the full consumption of compound **(13),** the catalyst was filtered over Celite and the crude product was concentrated under reduced pressure at RT. The acyl chloride was simultaneously concentrated under reduced pressure, dissolved in CH₂Cl₂ then the crude primary amine and 0.16 mL (0.916 mmol - 2.3 Eq.) of DIPEA were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced pressure. Purification by flash chromatography (100% EtOAc then CH₂Cl₂/EtOAc/MeOH 80/12/8 to 6/0/4) were conducted to separate the pure and impure fractions, to afford compound **(23)** (487.2 mg, 81%) as colorless oil.

### Analysis:

¹H NMR (400 MHz, MeOD) δ 7.24 (s, 2H), 6.85 (m, 3H), 4.22 - 4.20 (m, 4H), 4.16 (t, *J* = 5.7 Hz, 2H), 4.13 - 4.07 (m, 2H), 4.01 - 3.92 (m, 8H), 3.89 - 3.85 (m, 3H), 3.77 - 3.71 (m, 6H), 3.69 - 3.57 (m, 26H), 3.54 - 3.47 (m, 4H), 3.33 (s, 6H), 3.19 (ABx, J = 22.0 Hz, 4H), 1.66 - 1.55 (m, 8H), 1.29 (m, 78H), 0.90 (d, J = 7.3 Hz, 12H).

¹³C NMR (101 MHz, MeOD) δ 173.10, 169.67, 148.33, 141.71, 125.32, 116.02, 108.47, 72.97, 71.67, 71.58, 71.54, 71.36, 70.83, 70.03, 67.75, 67.67, 61.53, 59.10, 58.32, 40.71, 34.30, 33.12, 32.94, 31.67, 31.64, 31.61, 30.75, 30.73, 30.67, 30.52, 30.32, 26.70, 23.78, 20.86, 18.37, 14.50, 14.47.

³¹P NMR (162 MHz, MeOD) δ 27.16.

### 6.2. Step 2 - Preparation of compound (24)

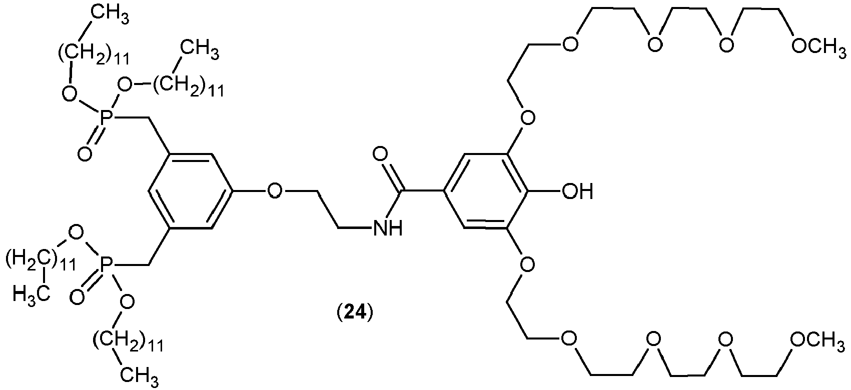

To a solution of 382 mg (0.236 mmol - 1.0 Eq.) of compound (23) in 40 mL of EtOAc were added 150.0 mg of Pd/C 10% as catalyst. The heterogeneous solution was purged with an atmosphere of hydrogen (five times), then vigorously stirred at RT for 5h, the catalyst was filtered off over Celite, the crude product was concentrated under reduced pressure, to give 320 mg of compound **(24)** as a colorless oil (89%).

### Analysis:

¹H NMR (400 MHz, MeOD) δ 7.24 (s, 2H), 6.85 (m, 3H), 4.25 - 4.18 (m, 4H), 4.16 (t, *J* = 5.7 Hz, 2H), 4.13 - 4.05 (m, 1H), 4.06 - 3.90 (m, 8H), 3.91 - 3.84 (m, 4H), 3.78 - 3.69 (m, 6H), 3.68 - 3.58 (m, 19H), 3.55 - 3.47 (m, 4H), 3.33 (s, 6H), 3.24 - 3.13 (ABx, J = 21.9 Hz, 4H), 1.59 (m, 8H), 1.37 - 1.20 (m, 78H), 0.90 (t, J = 6.85 Hz, 12H).

¹³C NMR (101 MHz, MeOD) δ 169.82, 148.27, 141.74, 125.30, 116.0, 108.40, 72.93, 71.71, 71.60, 71.53, 71.33, 70.77, 70.03, 67.74, 67.67, 61.53, 59.09, 58.35, 40.73, 34.27, 33.02, 31.66, 31.60, 30.67, 30.40, 30.27, 26.69, 23.74, 20.86, 18.39, 14.51, 14.46.

³¹P NMR (162 MHz, MeOD) δ 27.17.

### 6.3. Step 3 - Preparation of compound (25)

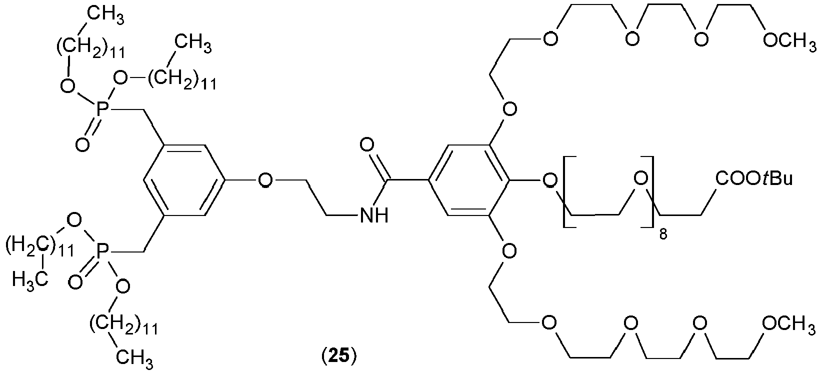

To a solution of 320 mg (0.209 mmol - 1.0 Eq.) of compound **(24)** in 40 mL of acetone, 0.145 g (0.219 mmol - 1.05 Eq.) of Linker 3 as prepared at step 6 of example 4, 33 mg (0.02 mmol - 0.1 Eq.) of KI and 45 mg (0.315 mmol - 1.5 Eq.) of K₂CO₃ were successively added. The resulting reaction mixture was heated to reflux overnight.

The reaction mixture was cooled to RT, the solvent was removed, the crude product was suspended in CH₂Cl₂, the solids were filtered off; the crude product was concentrated under reduced pressure to yield an orange oil. Purification by flash chromatography (CH₂Cl₂/MeOH 95/5 to 8/2 to 6/4) afforded 321 mg of compound **(25)** as a yellowish oil (76% yield).

### Analysis:

¹H NMR (400 MHz, MeOD) δ 7.27 (s, 2H), 6.86 (m, 3H), 4.30 - 4.22 (m, 6H), 4.18 (t, J = 5.6 Hz, 2H), 4.03 - 3.91 (m, 8H), 3.93 - 3.86 (m, 4H), 3.81 - 3.75 (m, 3H), 3.76 - 3.46 (m, 48H), 3.34 (s, 6H), 3.20 (ABx, J = 21.9 Hz, 4H), 2.48 (t, J = 6.2 Hz, 2H), 1.60 (m, 8H), 1.45 (s, 8H), 1.29 (s, 78H), 0.90 (t, *J* = 6.7 Hz, 12H).

¹³C NMR (101 MHz, MeOD) δ 153.63, 116.02, 107.32, 81.74, 72.95, 71.52, 71.46, 71.30, 70.53, 69.60, 67.86, 67.75, 59.15, 58.32, 37.19, 33.11, 31.67, 31.61, 30.75, 30.73, 30.51, 30.32, 28.40, 26.70, 23.77, 14.49.

³¹P NMR (162 MHz, MeOD) δ 27.16.

### 6.4. Step 4 - Preparation of a dendritic molecule of formula (I-F)

In a flask containing 321 mg (0.159 mmol - 1.0 Eq.) of compound (25) dissolved in DCM, 30 Eq. of TFA was added dropwise. The mixture was stirred for 2h, then evaporated under reduced pressure, affording 271mg of the dendritic molecule of formula **(I-F)** as a colorless oil (87%).

### Analysis:

¹H NMR (400 MHz, MeOD) δ 7.25 (s, 2H), 6.86 (m, 3H), 4.28 - 4.20 (m, 6H), 4.17 (t, *J* = 5.6 Hz, 2H), 3.97 (m, 8H), 3.92 - 3.85 (m, 4H), 3.82 - 3.73 (m, 4H), 3.76 - 3.53 (m, 45H), 3.55 - 3.48 (m, 4H), 3.33 (s, 6H), 3.20 (ABx, J = 21.9 Hz, 4H), 2.54 (t, J = 6.2 Hz, 2H), 1.67 - 1.53 (m, 8H), 1.31 (m, 78H), 0.90 (t, J = 7.0 Hz, 12H).

¹³C NMR (101 MHz, MeOD) δ 168.00, 152.33, 129.45, 114.59, 106.23, 72.25, 71.58, 70.22, 70.15, 70.02, 69.94, 69.86, 69.87, 69.23, 68.44, 66.39, 66.37, 66.25, 66.14, 57.69, 39.41, 34.37, 32.87, 31.59, 30.24, 30.21, 28.99, 28.85, 25.29, 22.33, 13.08.

³¹P NMR (162 MHz, MeOD) δ 27.16.

### EXAMPLE 7: Preparation of nanoemulsions according to the invention

### Materials and method

Nanoemulsions comprising a perfluorocarbon, a phospholipid and an oligo(ethylene glycol) (OEG) dendritic molecule of formula (I) according to the invention were prepared according to the following general procedure. The phospholipid and the dendron of formula (I) were dispersed by magnetic stirring in a buffer solution at a temperature higher than the phospholipid transition temperature. The perfluorocarbon was added dropwise to the dispersion and the mixture was pre-homogenized with a low energy device. The resulting dispersion was then submitted to sonication or high-pressure homogenization. After cooling down to room temperature, the dispersion was centrifuged and the transparent phase was filtrated on a 0.44 µm membrane. 1.5 mL-aliquots were taken and diluted with the buffer for size analysis by photosedimentation (e.g. Horiba-Capa 700) or dynamic light scattering (e.g. DLS, Malvern Zeta Sizer Nano ZS).
- Perfluorohexane (PFH, Fluorochem, Hadfield, UK),
- Perfluoropentane (PFP, Fluorochem, Hadfield, UK),
- Perfluorooctylbromide (Alliance Pharmaceutical, San Diego (CA, USA),
- Dipalmitoylphosphatidylcholine (DPPC): Avanti Polar Lipids,
- Dimyristoylphosphatidylcholine (DMPC): Avanti Polar Lipids,
- HEPES buffer (Sigma-Aldrich).

### Example 7.1: Preparation of a nanoemulsion comprising perfluorohexane (PFH), dipalmitoylphosphatidylcholine (DPPC) and a dendron of formula (I-P)

Dendron of formula (I-P) can be represented as follows:

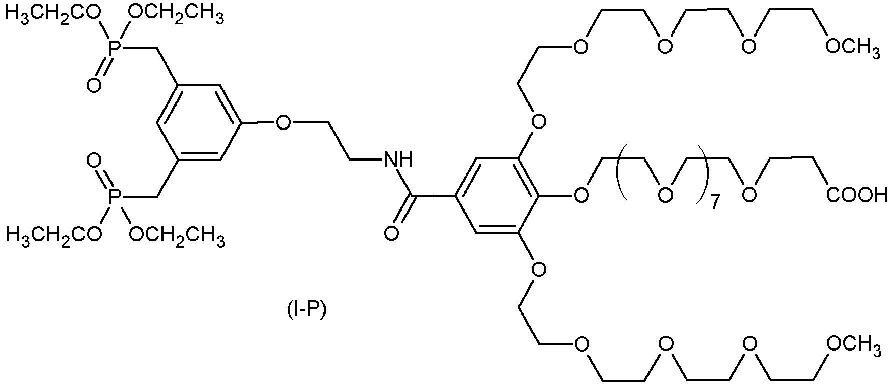

DPPC (9 × 10⁻³ mol L⁻¹, 6.60 g L⁻¹) and dendron of formula (I-P) (0.5 × 10⁻³ mol L⁻¹, 0.64 g L⁻¹) were dispersed by magnetic stirring for 2 h in 4 mL of HEPES buffer at 50°C. PFH (400 µL) was added dropwise to the dispersion, and the mixture was homogenized with a vortex mixer for 2 min. The resulting dispersion was submitted to tip sonication (Vibracell Sonicator, Bioblock Scientific, Illkirch) for 5 min at 25°C. After cooling to room temperature, the dispersion was centrifuged at 3000 rpm during 3 min and filtrated on a 0.44 µm membrane. The average diameter of the perfluorocarbon droplets in the resulting nanoemulsion was 170 ± 10 nm after preparation, as measured by DLS.

As a comparative example not forming part of the present invention, a reference nanoemulsion containing only DPPC and PFH but not the dendron of formula (I-P), prepared exactly in the same conditions, led to a coarser nanoemulsion (230 nm after preparation).

The average diameter of the perfluorocarbon droplets for each of these two prepared nanoemulsions was again measured by DLS at different times during a storage period of 2 months at 25°C (at t = 3 days, 10 days, 15 days, 30 days, 40 days and 60 days).

The results are reported on figures 1 and 2 annexed. Figure 1a corresponds to the droplet size distribution in the reference nanoemulsion stabilized by DPPC only and figure 1b corresponds to the droplet size distribution in the nanoemulsion according to the invention, i.e. stabilized by DPPC and dendron of formula (I-P). On figures 1a and 1b, intensity (%) is expressed as a function of diameter at t=0, i.e. just after the preparation of the nanoemulsions (dotted line curve) and after 2 months of storage at a temperature of 25°C (continuous line curve). Figure 2 shows the evolution of the average mean diameter of the droplets (in nm) as a function of time (in days) for each nanoemulsion. On figure 2, the curve with the black squares corresponds to the reference nanoemulsion stabilized by DPPC and the curve with the black disks corresponds to the nanoemulsion according to the invention, i.e. stabilized by DPPC and dendron of formula (I-P).

As it can be seen on these figures, the stability of a nanoemulsion comprising PFH droplets is greatly enhanced when said nanoemulsion is stabilized with both DPPC and a dendron of formula (I-P) comparatively to a reference nanoemulsion wherein the PFH droplets are stabilized only with DPPC. Indeed, the average diameter of the PFH droplet in the nanoemulsion according to the present invention did not change significantly after 2 months at 25°C (av. 190 ± 10 nm, Figure 1 b, and figure 2) while it changed significantly in the reference nanoemulsion with droplet size larger than 600 nm after 2 months at 25°C (Figure 1a, and Figure 2).

### Example 7.2: Preparation of a nanoemulsion comprising PFH, DPPC and a dendron of formula (I-P)

The above-described protocol of example 7.1 was applied to DPPC (9 × 10⁻³ mol L⁻¹, 6.60 g L⁻¹) and dendron of formula (I-P) (1 × 10⁻³ mol L⁻¹, 1.28 g L⁻¹). After addition of PFH (400 µL), homogenization by vortex mixer, and tip sonication followed by membrane filtration, a stable PFH nanoemulsion with a mean droplet diameter of 130 ± 10 nm (DLS) was obtained. The mean droplet diameter did not change significantly after 2 months at 25°C.

### Example 7.3: Preparation of a nanoemulsion comprising PFH, DPPC and a dendron of formula (I-P)

The above-described protocol of example 7.1 was applied to DPPC (4.5 × 10⁻³ mol L⁻¹, 3.30 g L⁻¹) and dendron of formula (I-P) (0.5 × 10⁻³ mol L⁻¹, 0.64 g L⁻¹). After addition of PFH (200 µL), homogenization by vortex mixer, and tip sonication followed by membrane filtration, a stable PFH nanoemulsion with a mean droplet diameter of 130 ± 8 nm (DLS) was obtained. The mean droplet diameter did not change significantly after 2 months at 25°C.

### Example 7.4: Preparation of a nanoemulsion comprising PFH, DPPC and a dendron of formula (I-Q)

Dendron of formula (I-P) can be represented as follows:

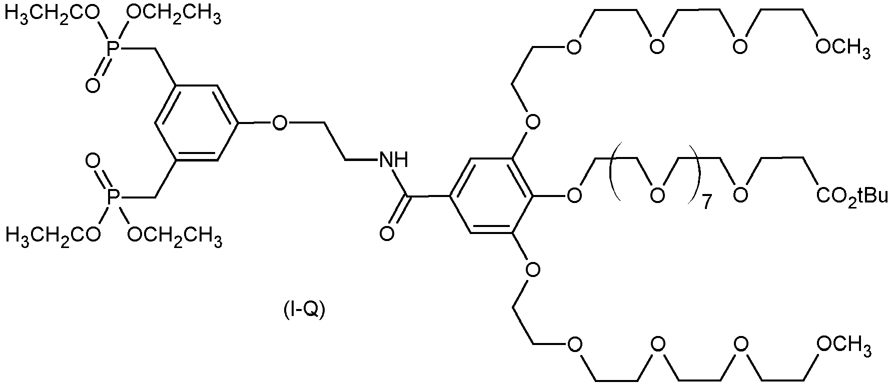

The above-described protocol of example 7.1 was applied to DPPC (9 × 10⁻³ mol L⁻¹, 6.60 g L⁻¹) and dendron of formula (I-Q) (1 × 10⁻³ mol L⁻¹, 1.45 g L⁻¹). After addition of PFH (400 µL), homogenization by vortex mixer, and tip sonication followed by membrane filtration, a stable PFH nanoemulsion with a mean droplet diameter of 145 ± 10 nm (DLS) was obtained. The mean droplet diameter did not change significantly after 2 months at 25°C.

### Example 7.5: Preparation of a nanoemulsion comprising PFH, DPPC and a dendron of formula (I-Q)

The above-described protocol of example 7.1 was applied to DPPC (9 × 10⁻³ mol L⁻¹, 6.60 g L⁻¹) and dendron of formula (I-Q) (0.5 × 10⁻³ mol L⁻¹, 0.72 g L⁻¹). After addition of PFH (400 µL), homogenization by vortex mixer, and tip sonication followed by membrane filtration, a stable PFH nanoemulsion with a mean droplet diameter of 185 ± 15 nm (DLS) was obtained. The mean droplet diameter did not change significantly after 2 months at 25°C.

### Example 7.6: Preparation of a nanoemulsion comprising perfluoropentane (PFP), DPPC and a dendron of formula (I-P)

The above-described protocol of example 7.1 was applied to DPPC (9 × 10⁻³ mol L⁻¹, 6.60 g L⁻¹) and dendron of formula (I-P) (1 × 10⁻³ mol L⁻¹, 1.28 g L⁻¹). After addition of PFP (400 µL), homogenization by vortex mixer, and tip sonication followed by membrane filtration, a stable PFP nanoemulsion with a mean droplet diameter of 250 ± 21 nm (DLS) was obtained. The mean droplet diameter did not change significantly after 2 months at 25°C.

### Example 7.7: Preparation of a nanoemulsion comprising PFP, DPPC and a dendron of formula (I-P)

The above protocol of example 7.1 was applied to DPPC (9 × 10⁻³ mol L⁻¹, 6.60 g L⁻¹) and dendron of formula (I-P) (0.5 × 10⁻³ mol L⁻¹, 0.64 g L⁻¹). After addition of PFP (400 µL), homogenization by vortex mixer, and tip sonication followed by membrane filtration, a stable PFP nanoemulsion with a mean droplet diameter of 202 ± 12 nm (DLS) was obtained. The mean droplet diameter did not change significantly after 2 months at 25°C.

### Example 7.8: Preparation of a nanoemulsion comprising PFP, DPPC and a dendron of formula (I-Q)

The above protocol of example 7.1 was applied to DPPC (9 × 10⁻³ mol L⁻¹, 6.60 g L⁻¹) and dendron of formula (I-Q) (0.5 × 10⁻³ mol L⁻¹, 0.72 g L⁻¹). After addition of PFP (400 µL), homogenization by vortex mixer, and tip sonication followed by membrane filtration, a stable PFP nanoemulsion with a mean droplet diameter of 202 ± 12 nm (DLS) was obtained. The mean droplet diameter did not change significantly after 2 months at 25°C.

### Example 7.9: Preparation of a nanoemulsion comprising perfluorooctylbromide (PFOB), DPPC and a dendron of formula (I-P)

The above-described protocol of example 1 was applied to DPPC (9 × 10⁻³ mol L⁻¹, 6.60 g L⁻¹) and dendron of formula (I-P) (1 × 10⁻³ mol L⁻¹, 1.28 g L⁻¹). After addition of PFOB (400 µL), homogenization by vortex mixer, and tip sonication followed by membrane filtration, a stable PFOB nanoemulsion with a mean droplet diameter of 90 ± 8 nm (DLS) was obtained. The mean droplet diameter did not change significantly after 2 months at 25°C.

### Example 7.10: Preparation of a nanoemulsion comprising PFOB, DPPC and a dendron of formula (I-Q)

The above-described protocol of example 7.1 was applied to DPPC (9 × 10⁻³ mol L⁻¹, 6.60 g L⁻¹) and dendron of formula (I-Q) (1 × 10⁻³ mol L⁻¹, 1.45 g L⁻¹). After addition of PFOB (400 µL), homogenization by vortex mixer, and tip sonication followed by membrane filtration, a stable PFOB nanoemulsion with a mean droplet diameter of 105 ± 10 nm (DLS) was obtained. The mean droplet diameter did not change significantly after 2 months at 25°C.

### Example 7.11: Preparation of a nanoemulsion comprising PFP, dimyristoylphosphatidylcholine (DMPC) and a dendron of formula (I-P)

The above-described protocol of example 7.1 was applied to DMPC (9 × 10⁻³ mol L⁻¹, 6.10 g L⁻¹) and dendron of formula (I-P) (0.5 × 10⁻³ mol L⁻¹, 0.64g L⁻¹). After addition of PFP (400 µL), homogenization by vortex mixer, and tip sonication followed by membrane filtration, a stable PFP nanoemulsion with a mean droplet diameter of 154 ± 8 nm (DLS) was obtained. The mean droplet diameter did not change significantly after 2 months 25°C.

### Example 7.12: Preparation of a nanoemulsion comprising PFH, dimyristoylphosphatidylcholine (DMPC) and a dendron of formula (I-P)

The above-described protocol of example 7.1 was applied to DMPC (3 × 10⁻² mol L⁻¹, 20.0 g L⁻¹) and dendron of formula (I-P) (1.2 × 10⁻³ mol L⁻¹, 1.5 g L⁻¹). After addition of PFH (400 µL), homogenization by vortex mixer, and tip sonication followed by membrane filtration, a stable PFH nanoemulsion with a mean droplet diameter of 257 ± 10 nm (DLS) was obtained.

### Example 7.13: Preparation of a nanoemulsion comprising PFH, dimyristoylphosphatidylcholine (DMPC) and a dendron of formula (I-P)

DMPC (3 × 10⁻² mol L⁻¹, 20.0 g L⁻¹) and dendron of formula (I-P) (1.2 × 10⁻³ mol L⁻¹, 1.5 g L⁻¹) were dispersed by magnetic stirring in 4 mL of HEPES buffer at room temperature for 2h. PFH (400 µL) was added dropwise to the dispersion at room temperature, and the mixture was homogenized with an IKA T10 Basic Ultra-Turrax mixer for 2 min. The resulting dispersion was then submitted to high-pressure homogenization (B15, Avestin, Canada) at 4 bars (6 cycles). The mean diameter of the perfluorocarbon droplets was 221 ± 10 nm, as measured by DLS.

As a comparative example not forming part of the present invention, a reference nanoemulsion containing only DMPC and PFH but not the dendron of formula (I-P), prepared exactly in the same conditions, led to a coarser nanoemulsion (270 nm after preparation).

The average diameter of the perfluorocarbon droplets for each of these two prepared nanoemulsions was again measured by DLS at different times during a storage period of 1 month at 25°C.

The results are reported of annexed figure 3.

On this figure, the mean diameter of the droplets (in nm) is expressed as a function of time (in days) for each nanoemulsion: the curve with the black squares corresponds to the reference nanoemulsion stabilized by DMPC and the curve with the black circles corresponds to the nanoemulsion according to the invention, i.e. stabilized by DMPC and dendron of formula (I-P).

As it can be seen on figure 3, the stability of a nanoemulsion comprising PFH droplets is greatly enhanced when said nanoemulsion is stabilized with both DMPC and a dendron of formula (I-P) comparatively to a reference nanoemulsion not forming part of the invention wherein the PFH droplets are stabilized only with DMPC. Indeed, the average diameter of the PFH droplets in the nanoemulsion according to the present invention are below 400 nm after 2 months at 25°C while it is above 550 nm after the same period of time.

### Example 7.14: Preparation of a nanoemulsion comprising PFH, dimyristoylphosphatidylcholine (DMPC) and a dendron of formula (I-D)

The protocol described in Example 7.13 was applied to DMPC (3 × 10⁻² mol L⁻¹, 20 g L⁻¹) and dendron of formula (I-D) as prepared above according to example 4 (1.2 × 10⁻³ mol L⁻¹, 1.93 g L⁻¹). After being submitted to high-pressure homogenization (B15, Avestin Canada, 4 bars, 6 cycles), the mean droplet diameter was 144 ± 10 nm and zeta potential was -30 mV. It is noteworthy that the zeta potential of a reference nanoemulsion not forming part of the present invention because containing only DMPC was -6 mV.

These results demonstrate that the stability of the nanoemulsion according to the invention is greatly superior than the stability of the reference nanoemulsion since it is well known that in a disperse system, the higher the zeta potential, the more the droplets tend to repel each other, which favors the stabilization of the nanoemulsion. In particular, as it is generally admitted and reported in the literature, a colloidal dispersion is considered as "stable" when its zeta potential is at least 30 mV (in absolute value).

### Example 7.15: Preparation of a nanoemulsion comprising PFH, dimyristoylphosphatidylcholine (DMPC) and a dendron of formula (I-D)

The protocol described in Example 7.13 was applied to DMPC (3 × 10⁻² mol L⁻¹, 20 g L⁻¹) and dendron of formula (I-D) as prepared above according to example 4 (1.2 × 10⁻³ mol L⁻¹, 1.87 g L⁻¹). After being submitted to high-pressure homogenization (B15, Avestin Canada, 6 bars, 6 passes), the mean droplet diameter was 231 ± 10 nm and zeta potential was -46.1 mV. The zeta potential of a reference nanoemulsion not forming part of the invention because formulated with DMPC only was -3.5 mV.

### Example 7.16: Preparation of a nanoemulsion comprising PFH, dimyristoylphosphatidylcholine (DMPC) and a dendron of formula (I-D)

The protocol described in Example 7.12 was applied to DMPC (3 × 10⁻² mol L⁻¹, 20 g L⁻¹) and dendron of formula (I-D) (1.9 × 10⁻³ mol L⁻¹, 3.2 g L⁻¹). After being submitted to high-pressure homogenization (B15, Avestin Canada, 6 bars, 6 cycles), the mean droplet diameter was 227 ± 10 nm and zeta potential was -16 mV.

### Example 7.17: Preparation of a nanoemulsion comprising PFH, dimyristoylphosphatidylcholine (DMPC) and a dendron of formula (I-E)

The protocol described in Example 7.12 was applied to DMPC (3 × 10⁻² mol L⁻¹, 20 g L⁻¹) and dendron of formula (I-E) as prepared above according to example 5 (1.2 × 10⁻³ mol L⁻¹, 2.1 g L⁻¹). After being submitted to high-pressure homogenization (B15, Avestin Canada, 6 bars, 6 cycles), the mean droplet diameter was 216 ± 10 nm and zeta potential was -25 mV.

As a comparative example not forming part of the present invention, a reference nanoemulsion containing only DMPC and PFH but not the dendron of formula (I-E), prepared exactly in the same conditions, led to a coarser nanoemulsion (260 nm after preparation).

The average diameter of the perfluorocarbon droplets for each of these two prepared nanoemulsions and also of the nanoemulsion prepared here above according to example 7.15 was again measured by DLS at different times during a storage period of 20 days at 25°C.

The results are reported of annexed figure 4.

On this figure, the mean diameter of the droplets (in nm) is expressed as a function of time (in days) for each nanoemulsion: the curve with black squares corresponds to the reference nanoemulsion stabilized by DMPC, the curve with black circles corresponds to the nanoemulsion of example 7.15 according to the invention stabilized by DMPC and dendron of formula (I-D) and the curve with black stars corresponds to the nanoemulsion according to the present example 7.16, i.e. stabilized by DMPC and dendron of formula (I-E).

As it can be seen on figure 4, the stability of the nanoemulsions comprising PFH droplets is greatly enhanced when said nanoemulsion is stabilized with both DMPC and a dendron of formula (I-D) or (I-E) comparatively to a reference nanoemulsion not forming part of the invention wherein the PFH droplets are stabilized only with DMPC. Indeed, the average diameter of the PFH droplets in the nanoemulsion according to the present invention did not change significantly after 10 days at 25°C while it changed significantly in the reference nanoemulsion with droplet size reaching 400 nm after 20 days at 25°C.

### Example 7.18: Preparation of a nanoemulsion comprising PFH, dimyristoylphosphatidylcholine (DMPC) and a dendron of formula (I-A)

DMPC (3 × 10⁻² mol L⁻¹, 20 g L⁻¹) and dendron of formula (I-A) prepared as described above in example 1 (1.2 × mol L⁻¹, 1.75 g L⁻¹) were dispersed by magnetic stirring in 4 mL of HEPES buffer at 25°C for 2h. PFH (400 µL) was added dropwise to the dispersion at 25°C, and the mixture was submitted to sonication. The mean diameter of the perfluorocarbon droplet was 550 ± 20 nm, as measured by DLS. The zeta potential was -2.1 mV.

### Example 7.19: Preparation of a nanoemulsion comprising PFH, dimyristoylphosphatidylcholine (DMPC) and a dendron of formula (I-B)

DMPC (3 × 10⁻² mol L⁻¹, 20.0 g L⁻¹) and dendron of formula (I-B) prepared as described above in example 2 (1.5 × mol L⁻¹, 1.75 g L⁻¹) were dispersed by magnetic stirring in 4 mL of HEPES buffer at 25°C for 2h. PFH (400 µL) was added dropwise to the dispersion at 25°C, and the mixture was submitted to sonication. The mean diameter of the perfluorocarbon droplet was 515 ± 8 nm, as measured by DLS. The zeta potential was -4.0 mV.

### EXAMPLE 8: Vaporization of perfluorocarbon nanoemulsions and control of microbubble size after activation

The following general method for phase-shifting perfluorocarbon nanoemulsion into stable microbubbles is described hereafter: A dendron-based perfluorocarbon nanoemulsion was activated into microbubbles by ultrasound or light irradiation. The microbubble mean diameter was determined immediately after preparation and monitored over time by optical microscopy and determination of the acoustic attenuation.

### Example 8.1: Phase shifting of a 10% v/v-concentrated PFP nanoemulsion.

The above-described protocol was applied the nanoemulsion prepared according to example 7.1 above, which is a 10% v/v-concentrated PFP nanoemulsion (mean diameter: 202 ± 12 nm), using two consecutive ultrasound pulses at 2.2 MHz and 1.1 MPa at 37°C.

Figure 5 and 6 annexed shows the mean diameter of the resulting microbubble dispersion (frequency (in %) as a function of the diameter of the microbubbles (in µm)) just after the preparation (figure 5) and after 7 hours at room temperature (figure 6).

Figure 7 is an optical micrograph of the resulting microbubble dispersion after 7 hours at room temperature.

The results presented in these figures show that the mean diameter of the resulting microbubble dispersion was 2.5 ± 0.7 µm after preparation, as assessed by optical microscopy and determination of the acoustic attenuation coefficient (figure 5). The microbubble mean diameter did not change significantly after 7 h at room temperature (figures 6 and 7) which demonstrates the excellent stability of the microbubble dispersion.

### Example 8.1: Phase shifting of a 5% v/v-concentrated PFP nanoemulsion.

The above-described protocol was applied the nanoemulsion prepared according to example 7.3 above, which is a 5% v/v-concentrated PFP nanoemulsion (mean diameter: 130 ± 8 nm), using two consecutive ultrasound pulses at 2.2 MHz and 1.1 MPa at 37°C. The microbubble mean diameter was 1.8 ± 0.6 µm after preparation, as assessed by optical microscopy and determination of the acoustic attenuation coefficient. The microbubble mean diameter did not change significantly after 7 h at room temperature.

### EXAMPLE 9: Evaluation of the anchorage of the dendron at the air/water interface of a microbubble dispersion

The anchoring of the dendrons in the microbubble shell was evaluated by investigating the isothermal compression of Langmuir monolayers formed by these molecules at the air/water interface. Langmuir monolayers constitute a bidimensional model of the shell of the nanoemulsions. In the above-mentioned experiments, the surface pressure (*π*) versus molecular area (A) isotherms were recorded using a Langmuir minitrough (KSV NIMA, Finland) equipped with two movable barriers (initial area: 365 × 75 mm², compression speed: 10 cm² min⁻¹, which corresponded to a reduction of the total area of ~3.6% min⁻¹). π was measured using the Wilhelmy plate (paper) method. The trough was maintained at 25 ± 0.5° C. Solutions of oligo (ethylene oxyde) dendrons (1 mmol L⁻¹) in chloroform were spread on the surface of water (320 mL). Subsequently, 15 min was allowed for chloroform to evaporate and the film to equilibrate before compression was initiated. All the experiments were performed at least three times. Since our Langmuir trough only allowed for a surface area compression of about 10, isotherms were recorded in three separate experiments.

These experiments have been performed using dendritic compounds of formulae (IA), (IB) and (IC) according to the present invention and also a comparative dendritic molecule not forming part of the present invention having the following formula DM:

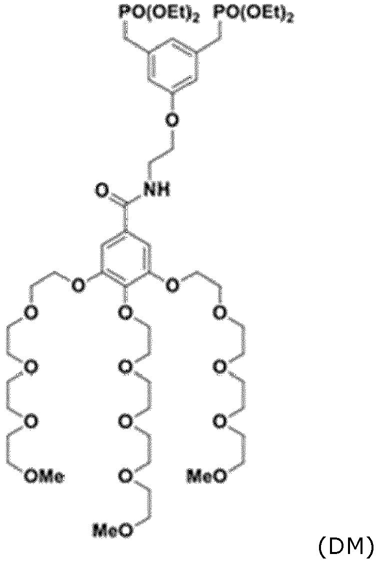

The results are reported on figure 8 annexed on which the surface pressure π (in mN m⁻¹) is expressed as a function of molecular area A (in Å²). On this figure, the continuous line curve corresponds to the experiment carried out using dendritic compound of formula (I-A), the curve with dashes corresponds to the experiment carried out using dendritic compound of formula (I-B), the curve with the dotted lines corresponds to the experiment carried out using dendritic compound of formula (I-C), and the curve with alternating dashes and dotted lines corresponds to the experiment carried out using dendritic compound of formula (DM) not forming part of the present invention.

These results show that grafting alkyl chains (dendron of formula I-A, dendron of formula I-B and dendron of formula I-C) on the bisphosphonate groups of the dendron strongly increased the stability of the monolayers, as demonstrated by much higher collapse pressures, e.g. ~45 mN m⁻¹ (Figure 8). For example, the stability, at the air/water interface, of a structurally-related dendron that does not contain long alkyl chains was low, exhibited a much lower surface pressure of ∼10 mN m⁻¹, revealing a weaker anchorage at the interface

## Claims

1. Use of an oligo(ethylene oxide) dendritic molecule of formula (I) below: 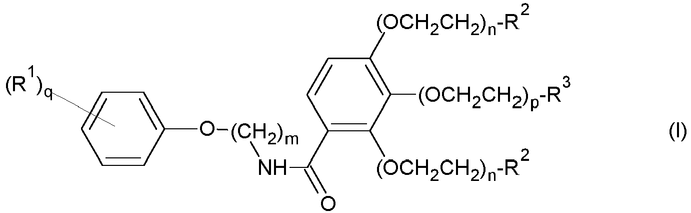 wherein :
- R¹ is a group selected among:
^{∗} an alkyl radical having at least 2 carbon atoms or an alkyl radical having at least 2 carbon atoms and comprising a terminal fluorinated group,
^{∗} a group -OR⁴ or -COOR⁴ in which R⁴ represents a linear alkyl radical having at least 4 carbon atoms or an alkyl radical having at least 2 carbon atoms and comprising a terminal fluorinated group, and
^{∗} a phosphonate group of the following formula (PG): 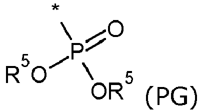 in which each of R⁵ represents a hydrogen atom or a linear alkyl radical having at least 1 carbon atom and the star represents the attachment point of said group of formula (PG) to the phenyl cycle;
- each of R² represents a linear alkyloxy radical having from 1 to 20 carbon atoms;
- R³ represents a linear alkyloxy radical having from 1 to 20 carbon atoms or a carboxyl group;
- n is an integer ranging from 1 to 16;
- p is an integer ranging from 1 to 16;
- m is an integer ranging from 1 to 4, preferably m = 1 or 2 and more preferably m = 2; and
- q is an integer ranging from 1 to 3, with the proviso that when R¹ represents a phosphonate group PG, then q = 2,
for stabilizing a fluorocarbon-based nanoemulsion consisting of a dispersion of nanodroplets of a liquid fluorocarbon stabilized in a continuous aqueous phase by a thin lipid film present at the interface between said aqueous phase and said liquid fluorocarbon, said lipid film comprising phospholipid(s) and at least one oligo(ethylene oxide) dendron of formula (I).

2. The use according to claim 1, wherein the oligo(ethylene oxide) dendritic molecule of formula (I) is a compound in which q = 2 and R¹ represents a phosphonate group of formula (PG) in which each of R⁵ represents a hydrogen atom or an alkyl group having from 4 to 12 carbon atoms.

3. The use according to claim 2, wherein said oligo(ethylene oxide) dendritic molecule of formula (I) are grafted on magnetic nanoparticles.

4. The use according to claim 1, wherein the oligo(ethylene oxide) dendritic molecule of formula (I) is a compound in which q = 2 and R¹ represents an alkyl group selected among octyl, decanyl, and dodecanyl or a fluorinated group selected among -(CH2)6-CF2CF3 and -(CH₂)₂-CF(CF₃)₂.

5. The use according to claim 1 wherein the oligo(ethylene oxide) dendritic molecule of formula (I) is a compound in which q = 2 and R¹ represents a group -OR⁴ or -COOR⁴ in which R⁴ represents an alkyl group selected among octyl, decanyl, and dodecanyl or a fluorinated group selected among -(CH2)6-CF2CF3 and -(CH₂)₂-CF(CF₃)₂.

6. The use according to any one of claims 1 to 5, wherein the oligo(ethylene oxide) dendritic molecule of formula (I) is a compound in which each of R² represents a methyloxy group.

7. The use according to any one of claims 1 to 6, wherein the oligo(ethylene oxide) dendritic molecule of formula (I) is a compound in which R³ represents a methyloxy group or a carboxyl group.

8. The use according to any one of claims 1 to 7, wherein the oligo(ethylene oxide) dendritic molecule of formula (I) is a compound selected among compounds of formulae (I-A) to (I-Q) whose significations of R¹ to R⁵, m, n p and q are given in the following Table 1:
**TABLE 1**
| **Cpds** | **R¹** | **R²** | **R³** | **R⁴** | **R⁵** | **n** | **p** | **m** | **q** |
|---|---|---|---|---|---|---|---|---|---|
| (I-A) | PG | -OCH₃ | -OCH₃ | - | -(CH₂)₇-CH₃ | 4 | 4 | 2 | 2 |
| (I-B) | PG | -OCH₃ | -OCH₃ | - | -(CH₂)₉-CH₃ | 4 | 4 | 2 | 2 |
| (I-C) | PG | -OCH₃ | -OCH₃ | - | -(CH₂)₁₁-CH₃ | 4 | 4 | 2 | 2 |
| (I-D) | PG | -OCH₃ | -COOH | - | -(CH₂)₇-CH₃ | 4 | 9 | 2 | 2 |
| (I-E) | PG | -OCH₃ | -COOH | - | -(CH₂)₉-CH₃ | 4 | 9 | 2 | 2 |
| (I-F) | PG | -OCH₃ | -COOH | - | -(CH₂)₁₁-CH₃ | 4 | 9 | 2 | 2 |
| (I-G) | -(CH₂)₇-CH₃ | -OCH₃ | -COOH | - | - | 4 | 9 | 2 | 2 |
| (I-H) | -(CH₂)₆CF₂CF₃ | -OCH₃ | -COOH | - | - | 4 | 9 | 2 | 2 |
| (I-I) | -(CH₂)₂CF(CF₃)₂ | -OCH₃ | -COOH | - | - | 4 | 9 | 2 | 2 |
| (I-J) | -COOR⁴ | -OCH₃ | -COOH | -(CH₂)₇-CH₃ | - | 4 | 9 | 2 | 2 |
| (I-K) | -COOR⁴ | -OCH₃ | -COOH | - (CH₂)₆CF₂C F₃ | - | 4 | 9 | 2 | 2 |
| (I-L) | -COOR⁴ | -OCH₃ | -COOH | - (CH₂)₂CF(C F₃)₂ | - | 4 | 9 | 2 | 2 |
| (I-M) | -OR⁴ | -OCH₃ | -COOH | -(CH₂)₇-CH₃ | - | 4 | 9 | 2 | 2 |
| (I-N) | -OR⁴ | -OCH₃ | -COOH | - (CH₂)₂CF(C F₃)₂ | - | 4 | 9 | 2 | 2 |
| (I-O) | -OR⁴ | -OCH₃ | -COOH | - (CH₂)₆CF₂C F₃ | - | 4 | 9 | 2 | 2 |
| (I-P) | PG | -OCH₃ | -COOH | -(CH₂)₇-CH₃ | H | 4 | 9 | 2 | 2 |
| (I-Q) | PG | -OCH₃ | -COO*t*Bu | | -CH₂CH₃ | 4 | 9 | 2 | 2 |

9. Fluorocarbon-based nanoemulsion comprising an aqueous continuous phase and a dispersion of nanodroplets consisting of a membrane of a lipid phase encapsulating at least one liquid fluorocarbon, wherein the lipid phase comprises at least one phospholipid and at least one oligo(ethylene oxide) dendritic molecule of formula (I) as defined in any one of claims 1 to 8.

10. The fluorocarbon-based nanoemulsion according to claim 9, wherein the at least one fluorocarbon is chosen among perfluorobutane, perfluoropentane, 2H,3H-perfluoropentane, perfluorohexane, perfluoroheptane, perfluorooctane, perfluorononane, perfluorodecalin, perfluorooctylbromide and perfluorotripropylamine.

11. The fluorocarbon-based nanoemulsion according to claim 9 or 10, wherein the concentration of fluorocarbon varies from 1 to 30% w/w.

12. The fluorocarbon-based nanoemulsion according to anyone of claims 9 to 11, wherein the at least one phospholipid is chosen among dimyristoylphosphatidylcholine, dipalmitoylphosphatidylcholine, and distearoylphosphatidylcholine.

13. The fluorocarbon-based nanoemulsion according to anyone of claims 9 to 12, wherein the concentration of the at least one phospholipid varies from 0.25 to 10% w/w.

14. The fluorocarbon-based nanoemulsion according to anyone of claims 9 to 13, wherein the concentration of the oligo(ethylene oxide) dendritic molecule of formula (I) varies from 0.07 to 0.7 % w/w.

15. The fluorocarbon-based nanoemulsion according to anyone of claims 9 to 14, wherein the phospholipid/oligo(ethylene oxide) dendritic molecule of formula (I) molar ratio varies from about 5:1 to 50:1.

16. A fluorocarbon-based nanoemulsion as defined in any one of claims 9 to 15, for its use in biomedical applications, in particular as contrast agents for echosonography or as an oxygenating agent for the treatment of hypoxia.
